# EUROPÄISCHE PATENTSCHRIFT

(11) **EP 2 363 108 B1**
(45) Veröffentlichungstag und Bekanntmachung des Hinweises auf die Patenterteilung: **30.08.2017**
(21) Anmeldenummer: 10155214.9
(22) Anmeldetag: 02.03.2010
(51) Int. Cl.: A61K 8/04, A61K 9/12, A61K 9/00

(54) **Schaumformulierungen enthaltend mindestens ein Triterpenoid**
Foam formulation containing at least one triterpenoid
Formulation d'une mousse contenant au moins un triterpénoïde

(43) Veröffentlichungstag der Anmeldung: 07.09.2011
(73) Patentinhaber: Neubourg Skin Care GmbH & Co. KG, 48268 Greven (DE)
(72) Erfinder: Neubourg, Thomas, 59368, Werne (DE)
(74) Vertreter: Jungblut & Seuss

(56) Entgegenhaltungen:
- WO-A1-01/72315
- WO-A1-03/026603
- WO-A1-2010/060896
- US-A1- 2006 057 075
- DANIELS R ET AL: "Skin care: Betulin for surfactant-free emulsions" PHARMAZEUTISCHE ZEITUNG 20080313 DE, Bd. 153, Nr. 11, 13. März 2008 (2008-03-13), Seiten 34-35, XP008124996 ISSN: 0031-7136

## Beschreibung

### Technisches Gebiet der Erfindung

Die vorliegende Erfindung betrifft kosmetische und dermatologische Schaumformulierungen, insbesondere Schaumcremes, auf Basis von Emulsionen, insbesondere vom Typ Öl-in-Wasser, wobei die Emulsion mindestens ein Triterpenoid umfasst.

### Hintergrund der Erfindung

### 1. Triterpenoide

Terpene sind Kohlenwasserstoffe natürlichen Ursprungs mit Kohlenstoffgerüsten, die sich formal von Isopren ableiten. Sie lassen sich unterteilen in Hemiterpene (C5-Gerüst), Monoterpene (C10-Gerüst), Sesquiterpene (C15-Gerüst), Diterpene (C20-Gerüst), Sesterpene (C25-Gerüst), Triterpene (C30-Gerüst) und Tetraterpene (C40-Gerüst).

Im Gegensatz dazu sind Terpenoide Naturprodukte und davon abgeleitete Substanzen, die auch Sauerstoff-haltige funktionale Gruppen enthalten können. Sie werden auf die gleiche Art wie bei Terpenen anhand der Zahl der Kohlenstoffatome des zugrundeliegenden Gerüsts eingeteilt. Triterpenoide basieren demnach auf einem C30-Gerüst.

Triterpenoide besitzen häufig ein tetrazyklisches oder pentazyklisches Grundgerüst. Pentazyklische Grundgerüste bestehend aus fünf annelierten Sechsringen umfassen beispielsweise das Oleanan-, das Friedelan- und das Ursan-Grundgerüst. Das pentazyklische Grundgerüst des Lupan-Typs hingegen beinhaltet neben vier Sechsringen einen Fünfring.

Beispielhafte Triterpenoide mit Oleanan-Grundgerüst sind Oleanolsäure, Erythrodiol, β-Amyrin, Glycyrrhetinsäure, und α-Boswelliasäure. Beispielhafte Triterpenoide mit Ursan-Grundgerüst sind Ursolsäure, α-Amyrin, β-Boswelliasäure und Corosolsäure. Ein beispielhaftes Triterpenoid mit Friedelan-Grundgerüst ist 2α,3β-Friedelandiol. Ein Lupan-Grundgerüst besitzen beispielsweise die Triterpenoide Betulin, Betulinsäure, Betulinsäuremethylester, Betulinaldehyd, Betulonsäure, Betulonaldehyd und Lupeol.

Triterpenoide können natürlichen oder synthetischen Ursprungs sein. Typischerweise werden sie durch Extraktion aus Pflanzenbestandteilen gewonnen. Extraktionsverfahren zur Isolierung von Triterpenoiden werden z.B. in den internationalen Anmeldungen WO 01/72315 A1 und WO 2004/016336 A1 beschrieben.

Der Birkenkork ist das triterpenoidreichste Gewebe der Pflanzenwelt (Hayek et al.; A bicentennial of Betulin, Phytochemistry 1989; 28: 2229-42). In geringeren Gehalten sind Triterpenoide auch in anderen Pflanzen weit verbreitet. So findet sich Betulinsäure in der Rinde der Platane (Galgon T. et al.; Phytochemical Analysis 1999; 10: 187-90.). Betulinsäure und Oleanolsäure sind in der Mistel enthalten (Jäger S et al., Planta Med 2007; 73:157-62), sowie im Rosmarin (Abe F et al., Biol Pharm Bull 2002; 25: 1485-7), der auch Ursolsäure enthält.

Es sind auch Wege bekannt, als Naturstoffe isolierte Triterpenoidgemische durch chemische Umsetzungen weiterzuverarbeiten, z.B. um eine Anreicherung bestimmter Triterpenoide zu bewirken. So beschreibt die WO 02/085921 A2 ein Verfahren zur Herstellung einer reinen Boswelliasäure aus einem Boswelliasäuregemisch.

Triterpenoide besitzen verschiedene pharmakologische Wirkungen. So wurden für Triterpenoide wie z.B. Betulin, Betulinsäure, oder Oleanolsäure antibakterielle, hepatoprotektive, antiplasmodiale, antivirale, antitumorale, wundheilungsfördernde, und antiinflammatorische Wirkungen beschrieben.

Auch für Boswelliasäuren, Inhaltsstoffe des Harzes des Weihrauchbaums Boswellia und des Myrrhebaums Commiphora, wurden entzündungshemmende, zytostatische und antitumorale Wirkungen beschrieben.

Die pharmakologischen Eigenschaften der Triterpenoide machen diese u.a. auch für dermatologische und kosmetische Anwendungen interessant. So zeigte eine Pilotstudie zur lokalen Behandlung aktinischer Keratosen mit einer Birkenkorkextrakt-haltigen Creme deren gute Wirksamkeit und Verträglichkeit (Huyke C. et al., Journal der Deutschen Dermatologischen Gesellschaft, 2006, 4 (2), Seiten 132-136). Ferner offenbart die WO 2005/123037 A1 Oleogele für kosmetischpharmazeutische Anwendungen, enthaltend eine unpolare Flüssigkeit und einen triterpenhaltigen Oleogelbildner. Gemäß der Offenbarung der WO 2005/123037 A1 liegt der Vorteil des Oleogels in der Einfachheit seiner Rezeptur, wobei das Triterpen gleichzeitig als pharmazeutisch wirksame Substanz und als Gelbildner fungiert.

Ferner offenbart DANIELS R ET AL in "Skin care: Betulin for surfactant-free emulsions"PHARMAZEUTISCHE ZEITUNG 20080313 DE, Bd. 153, Nr. 11, 13. März 2008 (2008-03-13), Seiten 34-35, eine Emulgatorfreie Emulsion Formulierung als Hautpflege umfassend eine Ölphase, eine Wasserphase und Betulin.

### 2. Emulsionen

Emulsionen stellen einen wichtigen Produkttyp im Bereich kosmetischer und/oder dermatologischer Zubereitungen dar, welcher in den unterschiedlichsten Anwendungsgebieten eingesetzt wird. Unter Emulsion versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (liphophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Der Begriff "Emulgator" bzw. "herkömmlicher Emulgator" ist im Stand der Technik bekannt. Herkömmliche Emulgatoren und ihre Verwendung werden beispielsweise in den Publikationen Pflegekosmetik, 4. Auflage, Wissenschaftliche Verlagsgesellschaft mbH Stuttgart, Seiten 151 bis 159, und Fiedler Lexikon der Hilfsstoffe, 5. Auflage, Editio Cantor Verlag Aulendorf, Seiten 97 bis 121, beschrieben.

Die IUPAC definiert den Begriff "Emulgator" wie folgt: "Emulgatoren sind grenzflächenaktive Substanzen. Sie halten sich bevorzugt in der Grenzfläche zwischen Öl- und Wasserphase auf und senken dadurch die Grenzflächenspannung. Emulgatoren erleichtern bereits bei niedriger Konzentration die Emulsionsbildung. Darüber hinaus vermögen diese Substanzen die Stabilität von Emulsionen zu verbessern, indem sie die Geschwindigkeit der Aggregation und/oder der Koaleszenz verringern."

Herkömmliche Emulgatoren können entsprechend ihrem hydrophilen Molekülteil in ionische (anionische, kationische und amphotere) Emulgatoren und nicht-ionische Emulgatoren untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nicht-ionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristika aller im System enthaltenen Stoffe zu berücksichtigen. Betrachtet man z. B. Hautpflegeemulsionen, so können polare Ölkomponenten, wie z. B. UV-Filter, zu Instabilitäten führen. Neben den Emulgatoren werden daher noch andere Stabilisatoren verwendet, die beispielsweise die Viskosität der Emulsion erhöhen und/oder als Schutzkolloid wirken.

### 3. Emulgatorfreie Emulsionen

Seit Jahrzehnten bilden herkömmliche Emulgatoren die Grundlage für die Entwicklung von Hautpflegepräparaten. Emulgatoren wurden als Hilfsstoffe zur Herstellung und vor allem zur Stabilisierung von Emulsionen eingesetzt. In jüngerer Zeit gibt es Hinweise, dass die Verwendung von Emulgatoren in Hautpflegepräparaten z.B. bei empfindlicher Haut zu Problemen führen kann, da die Emulgatoren typischerweise die Integrität der natürlichen Hautbarriere stören und es so bei der Hautreinigung zu einem Verlust von natürlichen Barrierestoffen der Haut kommen kann. Der Verlust der natürlichen Barrierestoffe kann erhöhte Rauigkeit, trockene Haut, Rissigkeit und Abnutzungsekzeme verursachen.

Weiterhin kann die Verwendung von Emulgatoren dazu führen, dass die lamellaren Strukturen der Lipidbarriere in vesikuläre Strukturen, wie z.B. Mizellen oder Mischmizellen umgewandelt werden. Diese Vesikel "zerstören" zumindest einen Teil der Barriereschicht der Haut und erhöhen dadurch lokal die Durchlässigkeit der Barriereschicht-Membran. Durch diese Öffnung der Barriereschicht der Haut wird zumindest vorübergehend der transepidermale Wasserverlust (TEWL) erhöht und gleichzeitig kommt es zu einer Abnahme des Feuchtigkeitsbindevermögens der Haut. Eine kontinuierliche Anwendung von Pflegepräparaten mit herkömmlichen Emulgatoren kann sogar dazu führen, dass die Haut nicht mehr in der Lage ist, ihre Schutzfunktion aufrecht zu erhalten.

Da herkömmliche Emulgatoren immer wieder als Ursache für Unverträglichkeiten bei Hautpflegeprodukten, wie z.B. eine Störung der Hautbarriere oder Mallorcaakne, genannt werden, sucht die kosmetische Industrie nach Alternativen zu den konventionellen Formulierungen in Form von emulgatorfreien Emulsionen. Emulgatorfreie Emulsionen sind frei von Emulgatoren im herkömmlichen Sinne, d.h. von amphiphilen Substanzen mit niedriger Molmasse (d.h. Molmasse < 5000 g/mol), die in geeigneten Konzentrationen Mizellen und/oder andere flüssigkristalline Aggregate ausbilden können.

Der Begriff "emulgatorfrei" ist im Fachgebiet etabliert. Nach einer im interdisziplinären Konsens zwischen Pharmazeuten, Dermatologen und anderen Fachleuten verabschiedeten Begriffsdefinition der Gesellschaft für Dermopharmazie (http://www.dermotopics.de/german/ausgabe_1_03_d/emulgatorfrei_1_2003_d.htm) kann eine Formulierung als "emulgatorfrei" bezeichnet werden, wenn sie statt mit Emulgatoren im engeren Sinne (d.h. herkömmlichen Emulgatoren) mit grenzflächenaktiven Makromolekülen (Molmasse von über 5000 g/mol) stabilisiert ist.

Als vielversprechender Ansatz für emulgatorfreie Emulsionen, mit dem Ziel, ausreichend stabile und kosmetisch ansprechende Produkte zu erhalten, die die mit herkömmlichen Emulgatoren verbundenen Nachteile vermeiden helfen, haben sich z.B. Feststoff- oder Polymer-stabilisierte Emulsionen erwiesen.

### 4. Feststoffstabilisierte Emulsionen

Ein Beispiel für emulgatorfreie Emulsionen sind Feststoff-stabilisierte Emulsionen. Feststoff-stabilisierte Emulsionen, die im Fachgebiet auch als Pickering-Emulsionen bezeichnet werden, sind durch feinstverteilte Feststoffteilchen stabilisiert und ermöglichen es, auf herkömmliche Emulgatoren weitestgehend zu verzichten.

In Feststoff-stabilisierten Emulsionen kommt es zu einer Anreicherung des festen Stoffes an der Phasengrenze Öl/Wasser in Form einer Schicht, wodurch ein Zusammenfließen der dispersen Phase verhindert wird.

Geeignete Feststoffemulgatoren sind insbesondere partikuläre, anorganische oder organische Feststoffe, die sowohl von hydrophilen als auch von lipophilen Flüssigkeiten benetzbar sind. Bevorzugt werden in den Feststoff-stabilisierten Emulsionen oder Pickering-Emulsionen z.B. Titandioxid, Zinkoxid, Siliziumdioxid, Fe₂O₃, Veegum, Bentonit oder Ethylcellulose als Feststoffemulgatoren eingesetzt.

In der EP 1 352 639 A1 bzw. der DE 101 62 840 werden Pickering-Emulsionen vorgestellt, die in Form von Lotionen, Cremes und Gelen zum Einsatz kommen. In der WO 2004/017930 sind weitere Pickering-Emulsionen beschrieben.

### 5. Polymer-stabilisierte Emulsionen

Ein weiteres Beispiel emulgatorfreier Emulsionen sind Polymer-stabilisierte Emulsionen. Bei Polymer-stabilisierten Emulsionen erfolgt die notwendige Stabilisierung im Gegensatz zu den klassischen Emulsionen nicht mit amphiphilen, tensidartigen Emulgatoren, sondern mit Hilfe geeigneter Makromoleküle. Derartig stabilisierte Formulierungen unterscheiden sich in ihrem Irritationspotenzial deutlich von den mit herkömmlichen Emulgatoren stabilisierten Emulsionen. Aufgrund ihrer hohen Molmasse können polymere Emulgatoren nicht in das Stratum corneum penetrieren. Unerwünschte Wechselwirkungen, z.B. im Sinne einer Mallorca-Akne, sind deshalb nicht zu erwarten.

Polymer-stabilisierte Emulsionen, insbesondere die ihnen zugrundeliegende Art der Stabilisierung, sowie geeignete Polymeremulgatoren sind in der europäischen Patentanmeldung mit der Anmeldungsnummer 09 015 330.5 und mit dem Titel "Emulgatorfreie, Polymer-stabilisierte Schaumformulierungen" des Anmelders Neubourg Skin Care GmbH & Co. KG im Kapitel "Polymer-stabilisierte Emulsionen" beschrieben. Der Inhalt der europäischen Patenanmeldung 09 015 330.5, insbesondere des Kapitels zu Polymer-stabilisierten Emulsionen (Seiten 6 bis 10), wird hiermit durch Verweis aufgenommen.

### 6. DMS-haltige Emulsionen

Es werden heute häufig Cremegrundlagen eingesetzt, die mit einer Vielzahl von natürlichen bzw. hautähnlichen Inhaltsstoffen arbeiten, die eine bessere Hautverträglichkeit insbesondere bei empfindlicher Haut versprechen. Dabei hat sich gezeigt, dass durch die Verwendung von hautähnlichen Inhaltsstoffen eine verbesserte Hautpflege erzielbar ist. So werden in diesen Cremegrundlagen z.B. einige Bestandteile der natürlichen Hautlipide wie z.B. Triglyceride durch (pflanzliche) Caprylsäure/Caprinsäuretriglyceride , Squalen durch (pflanzliches) Squalan, Ceramide durch Ceramide 3 (aus Hefe), Cholesterol durch (pflanzliche) Phytosterole und Phospholipide durch (pflanzliche) Phospholipide ersetzt.

Bevorzugt wird bei diesem Konzept auf viele übliche Hilfsstoffe wie Duftstoffe, Farbstoffe, komedogene Lipide (z.B. Mineralöle), Konservierungsmittel und physiologisch bedenkliche Emulgatoren verzichtet, da diese potenziell sensibilisierend sind und zu Irritationen der Haut führen können. Insbesondere werden diese Formulierungen bevorzugt ohne herkömmliche Emulgatoren zubereitet, um deren oben genannte Nachteile zu vermeiden.
Systeme, die auf spezifisch zusammengesetzte Membranlipide zurückgreifen, die eine lamellare Struktur der Membran aufweisen und damit die Struktur der physiologischen Haut-Lipid-Barriere nachempfinden, sind insbesondere unter dem Begriff "DMS®" (Derma Membrane Structure) in der Technik bekannt. Ein als "emulgatorfrei" bezeichnetes Handelsprodukt basierend auf einer DMS®-Grundlage wird beispielsweise unter dem Namen Physiogel® Creme vertrieben.

### 7. Triterpenoid-haltige Emulsionen

Aus der WO 01/72315 A1 sind Emulsionen bekannt, deren wässrige und fettige Phase durch einen Pflanzenextrakt emulgiert wird, wobei der Pflanzenextrakt wenigstens ein Triterpenoid und/oder wenigstens ein Derivat eines Triterpenoids aufweist. Das Triterpenoid und/oder sein Derivat soll die Emulsion konservieren und emulgieren und zudem pharmazeutisch aktiv sein. Dadurch wird gemäß der Offenbarung der WO 01/72315 A1 erreicht, dass die Emulsion keine zusätzlichen Konservierungsstoffe aufweist, wodurch sie einen besonders hohen Reinheitsgrad und eine gute Verträglichkeit vor allem bei problematischen Applikationen auf geschädigter Haut besitzt.

### 8. Schaumformulierungen

Eine besondere Anwendungsform kosmetischer und/oder dermatologischer Emulsionen ist die Applikation als Schäume. Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten. Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran, bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der *Perspiratio insensibiles* und keinem entsprechenden Wärmestau. Damit werden die positiven Eigenschaften einer Schutz- und Pflegefunktion mit einer unveränderten Hautatmung verbunden.

Schaumformulierungen enthalten häufig herkömmliche Emulgatoren, die für die Stabilisierung der Emulsion und für die darauf folgende Schaumstabilität sorgen. Letztere werden jedoch, wie bereits zuvor diskutiert, immer wieder als Ursache für Hautunverträglichkeiten genannt. Auf den Zusatz geeigneter Stabilisatoren kann allerdings nicht gänzlich verzichtet werden, da disperse Systeme, wie bereits beschrieben, beispielsweise Emulsionen thermodynamisch instabil sind.

In der WO 2008/138894 werden Schaumformulierungen auf Basis von emulgatorfreien Pickering-Emulsionen beschrieben.

Die WO 2008/155389 beschreibt Schaumformulierungen auf Basis von Emulsionen, deren Ölphase mindestens eine membranbildende Substanz umfasst, die in der Schaumformulierung lamellar angeordnete Membranen ausbildet, wobei die Emulsionen bevorzugt emulgatorfrei sind.

Es besteht jedoch weiterhin ein Bedarf an Hautpflegezusammensetzungen, die den Hautbedürfnissen gerecht werden und somit einen optimalen Hautschutz und eine optimale Hautpflege ermöglichen.

### Zusammenfassung der Erfindung

Die Anmelderin hat nun erkannt, dass sich triterpenoidhaltige Emulsionen als Basis für Schaumformulierungen eignen. Dabei werden die positiven Eigenschaften der Schaumformulierungen mit denen triterpenoidhaltiger Kosmetika verbunden. Es lassen sich insbesondere Schaumformulierungen ohne herkömmliche Emulgatoren oder mit sehr geringen Anteilen herkömmlicher Emulgatoren herstellen, die die positiven Eigenschaften des Schaumes, nämlich die physikalische Struktur und angenehme Anwendbarkeit, mit einer guten Hautverträglichkeit und ggf. Pflege- bzw. Heilwirkung verbinden. Dies macht solche Schaumformulierungen insbesondere verwendbar für kosmetische und dermatologische Formulierungen für empfindliche Hauttypen. Es wird dabei Verträglichkeit und Anwendungskomfort vorteilhaft miteinander verbunden.

Dabei ist es nicht selbstverständlich, dass das Verschäumen triterpenoidhaltiger Emulsionen zu stabilen Schaumprodukten führt. Schäume werden, wie bereits erwähnt, beispielsweise durch Einarbeiten von (druck)verflüssigten Treibgasen in O/W-Emulsionssysteme erhalten. Verdampft bei dem Verschäumen das in der dispersen Ölphase gelöste Treibgas, bildet sich ein Schaum (Gas-in-Flüssig-Dispersion). Beim Verdampfen bzw. Expandieren des in der dispersen Ölphase gelösten Treibgases kommt es zu einer Dilatation der dispersen Ölphase. Es ist nun überraschend gefunden worden, dass es beim Verschäumen der den erfindungsgemäßen Schaumformulierungen zugrundeliegenden Emulsionen nicht zum Brechen der Zubereitung kommt, und ein zur Anwendung in pharmazeutischen und kosmetischen Produkten geeigneter Schaum ausgebildet wird. Dieser ist stabil genug, um z.B. auf die Haut aufgetragen zu werden.

Insbesondere wurde überraschend gefunden, dass Emulsionen, die neben dem mindestens einen Triterpenoid noch weitere, insbesondere emulsionsstabilisierende, Inhaltsstoffe wie membranbildende Substanzen, Emulgator-Copolymere, und/oder Feststoffemulgatoren enthalten, besonders geeignete Grundlagen für Schaumformulierungen darstellen. So stellt beispielsweise eine Emulsion umfassend eine Ölphase und eine Wasserphase, die mindestens ein Triterpenoid umfasst, wobei die Ölphase mindestens eine membranbildende Substanz umfasst, die in der Schaumformulierung eine lamellar angeordnete Membran ausbildet, eine Grundlage für Schaumformulierungen dar, die zu besonders festen und cremigen Schäumen führt.

Ferner hat die Anmelderin erkannt, dass eine Emulsion umfassend eine Ölphase und eine Wasserphase, wobei die Emulsion mindestens ein Triterpenoid umfasst ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon, und wobei die Ölphase mindestens eine membranbildende Substanz umfasst, die in der Emulsion eine lamellar angeordnete Membran ausbildet, wobei die mindestens eine membranbildende Substanz ein Lipid umfasst, wobei das Lipid ein Phospholipid umfasst, eine besonders geeignete Grundlage für Schaumformulierungen darstellt.

Die Erfindung betrifft somit eine Schaumformulierung umfassend eine Emulsion, umfassend eine Ölphase und eine Wasserphase,
wobei die Emulsion mindestens ein Triterpenoid ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon, umfasst, und
wobei die Ölphase mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst.

Vorzugsweise handelt es sich bei der Schaumformulierung dabei um eine Schaumcreme.

Ferner betrifft die Erfindung die Verwendung der erfindungsgemäßen Schaumformulierungen als Träger für einen Wirkstoff, als Hautpflegemittel, als Hautreinigungsmittel, als Sonnenschutzmittel oder zur Herstellung eines Kosmetikums, Medizinprodukts oder Arzneimittels.

Ferner betrifft die Erfindung die Verwendung einer Emulsion, umfassend eine Ölphase und eine Wasserphase,
wobei die Emulsion mindestens ein Triterpenoid ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon umfasst, und
wobei die Ölphase mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst,
für die Herstellung einer Schaumformulierung.

Ferner betrifft die Erfindung die Verwendung mindestens eines Triterpenoids ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon, zur Stabilisierung von Schaumformulierungen umfassend eine Emulsion,
wobei die Ölphase der Emulsion mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst.

### Detaillierte Beschreibung der Erfindung

### 1. Definitionen

Gemäß der vorliegenden Erfindung sind Schaumformulierungen Formulierungen, insbesondere Emulsionen, die zur Erzeugung von Schaum sinnfällig hergerichtet sind. Die Formulierungen können insbesondere entweder mit (druck)verflüssigtem Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Beispielsweise können Pumpspraybehälter verwendet werden.

Gemäß der Erfindung sind herkömmliche Emulgatoren amphiphile Substanzen (aufweisend einen hydrophilen und einen lipohilen Molekülteil, die räumlich voneinander getrennt sind) mit einer Molmasse < 5000 g/mol, die an der Phasengrenze Öl/Wasser Grenzflächenfilme ausbilden.

Gemäß der Erfindung sind herkömmliche Emulgatoren amphiphile Substanzen mit einer Molmasse < 5000 g/mol, die in geeigneter Konzentration Mizellen und/oder andere flüssigkristalline Aggregate ausbilden können. Verbindungen oder Stoffgemische, die statt zur Mizellenbildung zur Ausbildung einer lamellar angeordneten Membran im Sinne der vorliegenden Erfindung führen, gelten jedoch nicht als herkömmliche Emulgatoren im Sinne der vorliegenden Erfindung.

Gemäß einem weiteren Aspekt der Erfindung sind herkömmliche Emulgatoren alle oberflächenaktiven Substanzen, die insbesondere bei üblichen Lagerungs- und Anwendungstemperaturen, wie z.B. Raumtemperatur, in der Emulsion nicht als Feststoff oder Polymer vorliegen.

Dies bedeutet, dass z.B. Fettalkohole mit einer Kettenlänge von 10 bis 40 Kohlenstoffatomen, soweit sie in einer Emulsion aufgrund deren Formulierung/ Zusammensetzung nicht als Feststoff, sondern beispielsweise flüssigkristallin oder gelöst vorliegen, unter die erfindungsgemäße Definition eines herkömmlichen Emulgators fallen. Liegen die Fettalkohole mit einer Kettenlänge von 10 bis 40 Kohlenstoffatomen in der Emulsion hingegen als Feststoff vor, so fallen sie nicht unter die erfindungsgemäße Definition eines herkömmlichen Emulgators.

Gemäß noch einem weiteren Aspekt der Erfindung sind herkömmliche Emulgatoren gemäß der vorliegenden Erfindung anionische, kationische, amphotere und nichtionische Tenside. Typische Vertreter der anionischen Tenside sind neutralisierte verzweigte und/oder unverzweigte, gesättigte oder ungesättigte Fettsäuren mit einer Kettenlänge von 10 bis 40 Kohlenstoffatomen. Typische Vertreter der kationischen Tenside sind Ammoniumverbindungen. Typische Vertreter der nichtionischen Tenside haben einen hydrophilen Molekülteil, wie etwa Glycerin, Polyglycerin, Sorbitan, Kohlenhydrate bzw. Polyoxyethlenglykole, der über Ester- und/oder Etherbindungen mit dem lipophilen Molekülteil verknüpft ist, der üblicherweise aus Fettalkoholen, Fettsäuren oder Isofettsäuren besteht. Zum Beispiel zählen die polyethoxylierten Fettsäureester mit Kettenlängen von 10 bis 40 Kohlenstoffatomen und mit einem Ethoxylierungsgrad von 5 bis 100 zu dieser Gruppe. Ferner zählen gesättigte und/oder ungesättigte, verzweigte und/oder unverzweigte Fettalkohole mit einer Kettenlänge von 10 bis 40 Kohlenstoffatomen zu den nichtionischen Emulgatoren. Häufig werden herkömmliche Emulgatoren in Kombinationen eingesetzt.

Triterpenoide fallen nicht unter die erfindungsgemäße Definition eines herkömmlichen Emulgators.

Gemäß der vorliegenden Erfindung sind im Wesentlichen emulgatorfreie Emulsionen solche Emulsionen, die weniger als 1,0 Gew.-% oder weniger als 0,5 Gew.-%, vorzugsweise weniger als 0,3 Gew.-%, mehr bevorzugt weniger als 0,1 Gew.-%, besonders bevorzugt weniger als 0,05 Gew.-% herkömmlicher Emulgatoren enthalten. Gemäß der Erfindung sind emulgatorfreie Emulsionen solche, die keine herkömmlichen Emulgatoren enthalten.

Gemäß der vorliegenden Erfindung ist ein Feststoffemulgator eine partikuläre Substanz, die sowohl von lipophilen als auch von hydrophilen Flüssigkeiten benetzbar ist. Es kann sich hierbei um anorganische oder organische Feststoffe handeln. Die Partikel können ferner unbehandelt oder beschichtet sein. Bevorzugt liegt die Partikelgröße zwischen 1 nm und 200 nm, mehr bevorzugt zwischen 5 nm und 100 nm. Werden organische Feststoffemulgatoren verwendet, wie beispielsweise kristalline Fettsäuren, kristalline Fettsäureester oder kristalline Fettalkohole, so kann die Partikelgröße auch zwischen 1 nm und 1000 nm liegen.

Gemäß der vorliegenden Erfindung liegt das mindestens eine Triterpenoid zumindest teilweise als partikulärer Feststoff vor, wenn von ungefähr 10 Gew.-% bis ungefähr 60 Gew.-% der eingesetzten Menge an Triterpenoid in der Emulsion als partikulärer Feststoff vorliegen.

Gemäß der vorliegenden Erfindung liegt das mindestens eine Triterpenoid im Wesentlichen als partikulärer Feststoff vor, wenn von ungefähr 60 Gew.-% bis ungefähr 100 Gew.-% der eingesetzten Menge an Triterpenoid in der Emulsion als partikulärer Feststoff vorliegen.

Gemäß der vorliegenden Erfindung ist eine lamellar angeordnete Membran so angeordnet, dass sie einen Schichtaufbau besitzt, derart, dass die jeweils obere Schicht der Substanz zu einer unteren Schicht der Substanz zueinander ausgerichtet ist. Die Ausrichtung der einzelnen Substanzschichten erfolgt zueinander unabhängig von dem verwendeten Lösungsmittel in der Art, dass z.B. die hydrophilen Reste der Substanz nach außen weisen und die hydrophoben Reste zueinander nach innen ausgerichtet sind oder umgekehrt.

Orientieren sich zwei Schichten der Substanz in dem oben beschriebenen Sinne, so spricht man von einer Einfachmembran, während bei einer Übereinanderordnung von zwei weiteren Schichten diese lamellare Struktur als Doppelmembran bezeichnet wird. Gemäß dem vorliegenden Prinzip können noch weitere Schichten an die bereits vorliegenden (Doppel)-Membran assoziiert werden, was zu einem Mehrfachmembranaufbau führt. Gemäß der vorliegenden Erfindung kann die Membran als Einfachmembran, als Doppelmembran oder auch als Mehrfachmembran vorliegen.

Unter dem Begriff einer freien Säure bzw. einer freien sauren funktionellen Gruppe sind gemäß der Erfindung Verbindungen mit einer Säurefunktion bzw. eine Säurefunktion zu verstehen, die zu mindestens 98%, bevorzugt mindestens 99% , besonders bevorzugt zu 100% nicht neutralisiert vorliegen.

Unter dem Begriff einer vollständig neutralisierten Säure bzw. einer vollständig neutralisierten sauren funktionellen Gruppe sind gemäß der Erfindung Verbindungen mit einer Säurefunktion bzw. eine Säurefunktion zu verstehen, die zu mindestens 98%, bevorzugt mindestens 99% , besonders bevorzugt zu 100% neutralisiert in Form ihrer Salze vorliegen.

Unter dem Begriff einer partiell neutralisierten Säure bzw. einer partiell neutralisierten sauren funktionellen Gruppe sind gemäß der Erfindung Verbindungen mit einer Säurefunktion bzw. eine Säurefunktion zu verstehen, die zu mindestens 2%, bevorzugt mindestens 1 %, und zu höchstens 98%, bevorzugt höchstens 99% neutralisiert in Form ihrer Salze vorliegen, während der nicht neutralisierte Teil als freie Säure vorliegt.

"Stabilisierung einer Schaumformulierung" kann im Rahmen der vorliegenden Erfindung bedeuten, dass die Bildung eines auf die Haut applizierbaren Schaumes ermöglicht wird, und/oder dass die Struktur des aus der Schaumformulierung gebildeten Schaumes länger aufrechterhalten werden kann, bevor der Schaum zerfällt, und/oder dass der aus der Schaumformulierung gebildete Schaum eine höhere Festigkeit aufweist. Gemäß einem weiteren Aspekt kann die Stabilisierung einer Schaumformulierung bedeuten, dass die Struktur des Schaumes für einen Zeitraum von mindestens 30 Sekunden, vorzugsweise mindestens 1 Minute, besonders bevorzugt mindestens 2 Minuten aufrechterhalten werden kann, bevor der Schaum zerfällt.

### 2. Zusammensetzung der Emulsion

Die den erfindungsgemäßen Schaumformulierungen zugrundeliegende Emulsion, die eine Ölphase und eine Wasserphase aufweist, umfasst mindestens ein Triterpenoid ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon. Dabei ist die Emulsion vorzugsweise eine Öl-in-Wasser Emulsion.

Die Emulsion umfasst neben dem mindestens einen Triterpenoid eine Ölphase, die mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst.

In einer weiteren Ausführungsform umfasst die Emulsion neben dem mindestens einen Triterpenoid mindestens ein grenzflächenaktives, ionisches Polymer mit einem Molekulargewicht von größer als 5000 g/mol, wobei das ionische Polymer ein Copolymer ist, das als Monomereinheiten ein ionisches Monomer (M1), und mindestens ein weiteres Monomer enthält (im Folgenden auch als "Emulgator-Copolymer" bezeichnet).

In noch einer weiteren Ausführungsform umfasst die Emulsion neben dem mindestens einen Triterpenoid mindestens einen Feststoffemulgator.

In weiteren bevorzugten Ausführungsformen umfasst die Emulsion neben dem mindestens einen Triterpenoid Kombinationen der soeben angegebenen weiteren Inhaltsstoffe. In diesen Ausführungsformen umfasst die Emulsion neben dem mindestens einen Triterpenoid:
aa) eine Ölphase, die mindestens eine membranbildende Substanz enthält, die in der Schaumformulierung eine lamellar angeordnete Membran ausbildet, und mindestens ein Emulgator-Copolymer; oder
bb) eine Ölphase, die mindestens eine membranbildende Substanz enthält, die in der Schaumformulierung eine lamellar angeordnete Membran ausbildet, und mindestens einen Feststoffemulgator; oder
cc) mindestens ein Emulgator-Copolymer und mindestens einen Feststoffemulgator; oder
dd) eine Ölphase, die mindestens eine membranbildende Substanz enthält, die in der Schaumformulierung eine lamellar angeordnete Membran ausbildet, und mindestens ein Emulgator-Copolymer und mindestens einen Feststoffemulgator.

Die obigen Ausführungsformen lassen sich dabei vorteilhaft mit allen in den folgenden Kapiteln a) bis k) beschriebenen, bevorzugten Ausführungsformen der Erfindung kombinieren.

### a) Triterpenoid

In einer bevorzugten Ausführungsform weist das mindestens eine Triterpenoid eine Löslichkeit in Wasser von kleiner als 0,5 % (m/m), mehr bevorzugt kleiner als 0,1 % (m/m), besonders bevorzugt kleiner als 0,05 % (m/m), ganz besonders bevorzugt kleiner als 0,01 % (m/m) auf. Die angegebenen Werte entsprechen der Löslichkeit gemessen bei Raumtemperatur.

In einer weiteren bevorzugten Ausführungsform weist das mindestens eine Triterpenoid eine Löslichkeit in Wasser von kleiner als 1 µg/mL, mehr bevorzugt kleiner als 0,75 µg/mL, besonders bevorzugt kleiner als 0,5 µg/mL, ganz besonders bevorzugt kleiner als 0,2 µg/mL auf. Die angegebenen Werte entsprechen der Löslichkeit gemessen bei Raumtemperatur.

Verfahren zur Bestimmung der Wasserlöslichkeit einer Substanz sind dem Fachmann bekannt. Insbesondere kann das in Jäger S. et al., Planta Med 2007; 73:157-62 und in Laszczyk, M., Triterpentrockenextrakt aus Birkenkork (Betula alba cortex), Untersuchungen zur chemischen Zusammensetzung, Galenik, Penetration und pharmakologisch-biologischen Wirkung, Dissertation, Albert-Ludwigs-Universität, Freiburg, 2007, Kapitel 3.8, beschriebene Verfahren zur Bestimmung der Wasserlöslichkeit von Triterpenoiden verwendet werden.

In einer bevorzugten Ausführungsform weist das mindestens eine Triterpenoid eine Löslichkeit in Pflanzenöl, vorzugsweise in raffiniertem Sonnenblumenöl, von kleiner als 50 mg/mL, mehr bevorzugt kleiner als 20 mg/mL, besonders bevorzugt kleiner als 10 mg/mL, ganz besonders bevorzugt kleiner als 8 mg/mL, kleiner als 5 mg/mL oder sogar kleiner als 3 mg/mL auf. Die angegebenen Werte entsprechen der Löslichkeit gemessen bei Raumtemperatur.

Verfahren zur Bestimmung der Löslichkeit einer Substanz in Ölen sind dem Fachmann ebenfalls bekannt. Insbesondere kann das in Laszczyk, M., Triterpentrockenextrakt aus Birkenkork (Betula alba cortex), Untersuchungen zur chemischen Zusammensetzung, Galenik, Penetration und pharmakologisch-biologischen Wirkung, Dissertation, Albert-Ludwigs-Universität, Freiburg, 2007, Kapitel 3.7, beschriebene Verfahren zur Bestimmung der Löslichkeit von Triterpenoiden in Pflanzenöl verwendet werden.

Vorzugsweise liegt das mindestens eine Triterpenoid in der Emulsion zumindest teilweise oder im Wesentlichen als partikulärer Feststoff vor. Dies weiß der Fachmann durch Auswahl des mindestens einen Triterpenoids, sowie dessen Menge in der Emulsion zu erreichen.

Vorzugsweise umfasst die Emulsion mindestens ein Triterpenoid ausgewählt aus der Gruppe bestehend aus tetrazyklischen und pentazyklischen Triterpenoiden oder Mischungen davon. Besonders bevorzugt umfasst die Emulsion mindestens ein pentazyklisches Triterpenoid.

In einer bevorzugten Ausführungsform umfasst die Emulsion mindestens ein Triterpenoid ausgewählt aus der Gruppe von pentazyklischen Triterpenoiden basierend auf einem Lupan-Grundgerüst, einem Ursan-Grundgerüst, einem Oleanan-Grundgerüst, einem Friedelan-Grundgerüst, oder Mischungen davon. Dabei sind die soeben genannten Verbindungen besonders bevorzugt die einzigen Triterpenoide in der Emulsion.

Besonders bevorzugt umfasst die Emulsion mindestens ein Triterpenoid ausgewählt aus der Gruppe bestehend aus Betulin, Betulinsäure, Lupeol, (C₁-C₆)Alkylestern der vorgenannten Säuren, Derivaten der vorgenannten Verbindungen, oder Mischungen davon. Dabei sind die soeben genannten Verbindungen vorzugsweise die einzigen Triterpenoide in der Emulsion.

Ganz besonders bevorzugt umfasst die Emulsion Betulin und/oder Derivate des Betulins, vorzugsweise Betulin.

Der Begriff "Derivate" gemäß der vorliegenden Erfindung umfasst dabei Verbindungen, die einen oder mehrere zusätzliche Substituenten an dem Triterpenoid-Gerüst, Doppelbindungen anstelle von Einzelbindungen, geänderte Stereochemie oder relokalisierte Methyl- und/oder Isopropylgruppen aufweisen; sowie (C₁-C₈)-Alkylester und/oder (C₁-C₈)-Alkylether der Verbindungen.

Die zusätzlichen Substituenten werden dabei vorzugsweise ausgewählt aus der Gruppe bestehend aus (C₁-C₈)-Alkyl, (C₁-C₈)-Alkenyl, (C₁-C₈)-Alkinyl, (C₁-C₈)-Hydroxyalkyl, (C₁-C₈)-Hydroxyalkenyl, (C₁-C₈)-Hydroxyalkinyl, (C₁-C₈)-Alkyloxy, (C₁-C₈)-Alkylcarbonyl, (C₁-C₈)-Alkyloxycarbonyl, (C₁-C₈)-Alkylcarboxy, -COOH-Gruppen, Hydroxy-Gruppen und Keto-Gruppen; wobei die Alkyl-, Alkenyl-, und Alkinylgruppen linear oder verzweigt sein können.

In einer weiteren bevorzugten Ausführungsform umfasst die Emulsion mindestens ein Triterpenoid ausgewählt aus der Gruppe von pentazyklischen Triterpenoiden basierend auf einem Lupan-Grundgerüst, einem Ursan-Grundgerüst, einem Oleanan-Grundgerüst, einem Friedelan-Grundgerüst, oder Mischungen davon, wobei das mindestens eine Triterpenoid 1 bis 5 Hydroxy-Substituenten, bevorzugt 1 bis 3 Hydroxy-Substituenten, besonders bevorzugt 1 bis 2 Hydroxy-Substituenten umfasst, oder das mindestens eine Triterpenoid 1 bis 3 Hydroxy-Substituenten und 1 bis 3 COOH-Substituenten, bevorzugt 1 bis 2 Hydroxy-Substituenten und 1 bis 2 COOH-Substituenten umfasst.

Die Emulsion umfasst vorzugsweise mindestens 0,05 Gew.-% des mindestens einen Triterpenoids, mehr bevorzugt mindestens 0,08 Gew.-% des mindestens einen Triterpenoids, besonders bevorzugt mindestens 0,1 Gew.-% des mindestens einen Triterpenoids, und ganz besonders bevorzugt mindestens 0,2 Gew.-% des mindestens einen Triterpenoids. Zum Beispiel kann die Emulsion von ungefähr 0,05 Gew.-% bis ungefähr 10 Gew.-% des mindestens einen Triterpenoids, bevorzugt von ungefähr 0,08 Gew.-% bis ungefähr 8 Gew.-% des mindestens einen Triterpenoids, mehr bevorzugt von ungefähr 0,1 Gew.-% bis ungefähr 5 Gew.-% des mindestens einen Triterpenoids und ganz besonders bevorzugt von ungefähr 0,2 Gew.-% bis ungefähr 3 Gew.-% des mindestens einen Triterpenoids umfassen. Die obigen Angaben beziehen sich dabei jeweils auf das Gesamtgewicht der Emulsion ohne Treibgas.

In einer bevorzugten Ausführungsform umfasst die Emulsion mindestens 0,05 Gew.-% Betulin, mehr bevorzugt mindestens 0,08 Gew.-% Betulin, besonders bevorzugt mindestens 0,1 Gew.-% Betulin, und ganz besonders bevorzugt mindestens 0,2 Gew.-% Betulin. Zum Beispiel kann die Emulsion von ungefähr 0,05 Gew.-% bis ungefähr 10 Gew.-% Betulin, bevorzugt von ungefähr 0,08 Gew.-% bis ungefähr 8 Gew.-% Betulin, mehr bevorzugt von ungefähr 0,1 Gew.-% bis ungefähr 5 Gew.-% Betulin und ganz besonders bevorzugt von ungefähr 0,2 Gew.-% bis ungefähr 3 Gew.-% Betulin umfassen. Die obigen Angaben beziehen sich dabei jeweils auf das Gesamtgewicht der Emulsion ohne Treibgas.

Das mindestens eine Triterpenoid kann beispielsweise in Form mindestens eines triterpenoidhaltigen Pflanzenextrakts vorliegen.

Geeignete Pflanzenextrakte umfassen Birkenkorkextrakte, Extrakte aus der Rinde von Betula alba, Extrakte aus der Rinde von Betula pendula, Extrakte aus der Rinde von Betula platyphylla, Extrakte aus dem Harz des Weihrauchbaums Boswellia, Extrakte aus dem Harz des Myrrhebaums Commiphora, Extrakte aus der Rinde der Platane, Mistelextrakte, Rosmarinextrakte, Extrakte aus den Wurzeln und/oder Blättern der Weiß-Esche Fraxinus americana, und Extrakte aus den Blättern und/oder der Rinde der Eberesche Sorbus americana.

Besonders bevorzugt liegt das Triterpenoid in Form mindestens eines triterpenoidhaltigen Pflanzenextrakts vor, der vorzugsweise ausgewählt ist aus der Gruppe bestehend aus Birkenkorkextrakten, Extrakten aus der Rinde von Betula alba, Extrakten aus der Rinde von Betula pendula, Extrakten aus der Rinde von Betula platyphylla, oder Mischungen davon.

Liegt das mindestens eine Triterpenoid in Form eines Pflanzenextrakts vor, umfasst dieser vorzugsweise mindestens 40 Gew.-% Betulin, mehr bevorzugt mindestens 50 Gew.-%, mindestens 60 Gew.-% oder mindestens 70 Gew.-% Betulin (jeweils bezogen auf das Gesamtgewicht des Pflanzenextrakts). Ein besonders geeigneter triterpenoidhaltiger Pflanzenextrakt ist dabei beispielsweise der Extrakt aus der äußeren Rinde von Betula Alba, Betula platyphylla oder Betula pendula.
Ein geeigneter Extrakt aus der Rinde von Betula Alba ist beispielsweise erhältlich von der Firma Rita Corporation, Crystal Lake, USA, unter der Bezeichnung "Ritalab White Birch Bark Extract P" (CAS-Nummer 84012-15-7) in Form eines weißen bis cremefarbenen Pulvers mit einem Schmelzbereich von 238 °C bis 256 °C. Dieser Extrakt enthält ca. 70 Gew.-% Betulin, sowie 4-10 Gew.-% Betulinsäure.

Ein weiterer geeigneter triterpenoidhaltiger Pflanzenextrakt ist erhältlich von der Firma Aromtech, Kiviranta, Finnland, unter der Bezeichnung "Melabel Arctic Birch Bark Extract" (CAS-Nr. 85940-29-0). Dieser wird durch ethanolische Extraktion der äußeren Rinde von Betula Alba und Betula pendula gewonnen und liegt in Form eines hellbeige-farbenen Pulvers vor. Dieses enthält mindestens 45 Gew.-% Betulin und mindestens 3 Gew.-% Lupeol.

### b) Ölphase

Geeignete Komponenten, welche die Ölphase bilden können, können aus polaren und unpolaren Lipiden oder deren Mischungen gewählt werden. Die Ölphase umfasst erfindungsgemäß mindestens eine membranbildende Substanz, die in der Schaumformulierung eine lamellar angeordnete Membran ausbildet, wobei die mindestens eine membranbildende Substanz ein Lipid umfasst, wobei das Lipid ein Phospholipid umfasst.

Das Phospholipid ist etwa ein Lecithin. Weitere Lipide die auch die Ölphase bilden können, sind (Mono-, Di-, Tri-)-Glyceride (insbesondere Triglyceride, wie z.B. der Fettsäuretriglyceride), Sphingolipide, aus der Gruppe der Propylenglykol- oder Butylenglykol-Fettsäureester, aus der Gruppe der natürlichen Wachse, tierischen und pflanzlichen Ursprungs, aus der Gruppe der Esteröle, aus der Gruppe der Dialkylether und Dialkylcarbonate, aus der Gruppe der verzweigten und unverzweigten Kohlenwasserstoffe und Wachse sowie aus der Gruppe der zyklischen und linearen Silikonöle.

In einer Ausführungsform umfasst die Ölphase mindestens einen Fettsäurealkylester wie beispielsweise Ölsäuredecylester (Decyloleat) oder Cetearylisononanoat, und/oder mindestens einen Fettalkohol wie beispielsweise 2-Octyldodecanol. Ferner kann die Ölphase gesättigte aliphatische Kohlenwasserstoffe wie Paraffin enthalten.

Decyloleat ist beispielsweise von der Fa. Cognis unter dem Namen Cetiol V erhältlich. Cetearylisononanoat ist beispielsweise von der Fa. Cognis unter dem Namen Cetiol SN erhältlich. 2-Octyldodecanol ist beispielsweise von der Fa. Cognis unter dem Namen Eutanol G erhältlich.

In einer bevorzugten Ausführungsform umfasst die Ölphase mindestens ein Triglycerid. Bevorzugt umfasst das mindestens eine Triglycerid Caprylsäure-/Caprinsäuretriglycerid, erhältlich unter der Bezeichnung Miglyol 812 der Firma Sasol, und dessen Abmischung mit weiteren Öl- und Wachskomponenten. Besonders bevorzugt sind weiterhin Triglyceride, insbesondere Caprylsäure-/Caprinsäuretriglycerid, erhältlich unter der Bezeichnung Miglyol 812 der Firma Sasol/Myritol 312 der Fa. Cognis.

Außerdem kann die Ölphase bevorzugt weitere Bestandteile wie z.B. Fettsäuren, insbesondere Stearinsäure enthalten. Die Ölphase kann vorzugsweise pflanzliche Öle, wie beispielsweise Mandelöl, Avocadoöl, Sonnenblumenöl, oder Olivenöl enthalten.

Die erfindungsgemäßen Emulsionen enthalten bevorzugt von 5 bis 50 Gew.-% Ölphase, besonders bevorzugt 10 bis 35 Gew.-% und mehr bevorzugt 15 bis 35 Gew.-% Ölphase. Die Angaben werden jeweils auf das Gesamtgewicht der Emulsion ohne Treibgas bezogen.

### c) Membranbildende Substanzen in der Ölphase

Die Ölphase der Emulsion umfasst gemäß Anspruch 1 mindestens eine membranbildende Substanz, die in der Schaumformulierung eine lamellar angeordnete Membran ausbildet. Bei der mindestens einen membranbildenden Substanz kann es sich um einzelne Verbindungen oder um ein Substanzgemisch, vorzugsweise um ein Substanzgemisch handeln.

Vorzugsweise weisen die membranbildenden Substanzen der Erfindung einen HLB-Wert von größer als 8 auf, mehr bevorzugt von 9 bis 11, und am meisten bevorzugt von 9,5 bis 10,5.

Die membranbildenden Substanzen und Substanzgemische gemäß der vorliegenden Erfindung sind typischerweise in Wasser unlöslich, während herkömmliche Emulgatoren, insbesondere Tenside mit einem vergleichbaren HLB-Wert von etwa 10, in der Regel wasserlöslich sind. Ferner sind die wasserunlöslichen membranbildenden Substanzen gemäß der vorliegenden Erfindung nicht in der Lage, spontan Öle zu emulgieren, während herkömmliche Emulgatoren, insbesondere jene mit hohem HLB-Wert, spontan Öle emulgieren können. Herkömmliche Emulgatoren mit niedrigem HLB-Wert sind im Gegensatz zu membranbildenden Substanzen gemäß der vorliegenden Erfindung, und zwar Phospholipiden, nicht in der Lage, alleine lamellare Strukturen oder Liposomen zu bilden.

Membranbildende Substanzen im Sinne der vorliegenden Erfindung bilden normalerweise alleine keine Mizellen oder hexagonale flüssigkristalline Phasen. Sie bilden oberhalb der Phasenübergangstemperatur in Wasser spontan ausschließlich große multilamellare Vesikel (large multilamellar vesicles). Unterhalb der Phasenübergangstemperatur lassen sie sich unter hohem Energieeintrag in Wasser dispergieren und bilden lamellare Strukturen. Die genannte Phasenübergangstemperatur gibt hierbei die Temperatur an, bei der eine gelförmige Phase in eine flüssigkristalline Phase übergeht. Unterhalb der Phasenübergangstemperatur liegt eine Gelphase vor, oberhalb davon liegt eine flüssigkristalline Phase vor. Die Phasenübergangstemperaturen variieren je nach Zusammensetzung (gesättigt/ungesättigt; kurz/lang) und liegen beispielsweise bei Phospholipiden typischerweise zwischen 10°C und 70°C. Für ein gegebenes System kann die Phasenübergangstemperatur leicht mittels DSC gemessen werden.

Gemäß der vorliegenden Erfindung ist eine membranbildende Substanz, die eine lamellar angeordnete Membran ausbildet, eine Substanz, die vorzugsweise zugleich einen hydrophilen sowie einen hydrophoben Molekülrest aufweist. Die Fähigkeit einer membranbildenden Substanz, statt Mizellen lamellare Strukturen zu bilden, hängt aber insbesondere von der optimalen Fläche aₒ (Grenzfläche Kohlenstoff/Wasser), dem Volumen V und der kritischen Kettenlänge l_{c} ab (Israelachvili, Jacob N.: "Intermolecular and Surface Forces: With Applications to Colloidal and Biological Systems". 2nd Edition Academic Press, London, UK, 1992). Weiterhin ist es unter Umständen notwendig, spezielle Herstellungsbedingungen zu wählen, damit ein System lamellare Strukturen ausbildet. Geeignete Bedingungen werden weiter unten beschrieben.

Erfindungsgemäß umfasst die Ölphase ein Lipid, das ein Phospholipid, z.B. ein Lecithin, umfasst. Weitere Beispiele für membranbildende Substanzen sind z.B. Sphingolipide, Ceramide, Cholesterin, Fettalkohole, Fettsäuren sowie deren Mono- und/oder Diester, sowie Sterole. Triglyceride (nicht hydrophil und lipophil), Squalen (nicht hydrophil und lipophil), Squalan (nicht hydrophil und lipophil), können ebenfalls in Stoffgemischen enthalten sein, die membranbildende Substanzen umfassen.

Solche Substanzen oder entsprechende Substanzgemische können auf geeignete Weise mit einer wässrigen Phase unter Ausbildung von lamellaren Membranen dispergiert werden. Dies kann beispielsweise durch Dispergieren unter hohem Energieeintrag (z.B. Hochdruckhomogenisation, Ultraschall) erreicht werden, wie weiter unten beschrieben. Die Verwendung von bestimmten, kommerziell erhältlichen Fertigkonzentraten, die eine lamellare Phase ausbilden, ist ebenfalls möglich.

In einer bevorzugten Schaumformulierung der Erfindung umfasst das Phospholipid Lecithin, vorzugsweise hydriertes Lecithin.

Vorzugsweise umfasst die Emulsion ferner weitere Bestandteile, etwa Stabilisatoren wie z.B. Alkohole oder Glykole. Bevorzugt sind dabei Glykole, insbesondere Propylenglykol, Caprylylglykol oder Mischungen davon.

In weiteren bevorzugten Ausführungsformen umfasst die Emulsion weitere Bestandteile wie z.B. Butyrospermum Parkii (Sheabutter), Squalan, Glyceride, Ceramide, bevorzugt Ceramid 3 oder Mischungen der vorgenannten.

Gemäß der vorliegenden Erfindung wird die Ölphase, die eine zur Bildung von lamellar angeordneten Membranen geeignete Substanz oder ein solches Substanzgemisch umfasst, mit einer Wasserphase unter Bedingungen dispergiert, die zur Bildung einer lamellaren Phase führen. Falls notwendig geschieht dies beispielsweise durch Dispergieren unter hohem Energieeintrag, wie z.B. unter Ultraschall oder mittels Hochdruckhomogenisation, wobei Drücke von etwa 50.000 bis etwa 250.000 Kilopascal (etwa 500 bis etwa 2500 bar), vorzugsweise etwa 100.000 bis etwa 150.000 Kilopascal (etwa 1000 bis etwa 1500 bar) verwendet werden. In anderen Fällen, insbesondere bei Verwendung von nicht-hydriertem Lecithin mit niedriger Phasenübergangstemperatur als membranbildende Substanz, reicht häufig bereits einfaches Dispergieren aus, ohne dass zusätzlich ein hoher Energieeintrag notwendig wäre.

Das Vorliegen von lamellaren Strukturen in der Dispersion kann der Fachmann leicht mit Hilfe im Stand der Technik bekannter Verfahren feststellen. Geeignete Messmethoden sind beispielsweise in Claus-Dieter Herzfeld et al. (Hrsg.), Grundlagen der Arzneiformlehre, Galenik 2, Springer Verlag, 1999, beschrieben. Besonders erwähnenswert ist hierbei die Methode der Polarisationsmikroskopie.

Dabei werden zwei Polarisationsfolien in der sogenannten Kreuzstellung, bei der die Schwingungsebenen des erzeugten polarisierten Lichts senkrecht zueinander stehen über und unter dem zu untersuchenden Objekt angebracht. Die Schwingungsebene des eingestrahlten Lichts wird durch die Probe verändert, so dass ein Anteil des Lichts die zweite Polarisationsfolie passieren kann. Das Vorliegen von Lamellarphasen ist hier typischerweise durch sogenannte Malteserkreuze erkennbar.

In einer besonders bevorzugten Ausführungsform umfasst die membranbildende Substanz ein Phospholipid, wie etwa Lecithin oder hydriertes Lecithin, und zusätzlich ein weiteres Lipid. Mehr bevorzugt ist das Phospholipid ein Gemisch aus Lecithin und hydriertem Lecithin. In einer besonders bevorzugten Ausführungsform beträgt dabei das Gewichtsverhältnis von Lecithin zu hydriertem Lecithin etwa 10 : 1 bis etwa 1 : 10, mehr bevorzugt etwa 5 : 1 bis etwa 1 : 5 und noch mehr bevorzugt beträgt das Verhältnis von Lecithin zu hydriertem Lecithin etwa 1 : 1. Das zusätzlich zum Phospholipid vorhandene Lipid umfasst in einer bevorzugten Ausführungsform einen flüssigen Wachsester, wie etwa Isopropylmyristat, -palmitat, -stearat oder dergleichen. Ferner können optional weitere Lipide, wie etwa Erdnussöl oder mittelkettige Triglyceride (bevorzugt C₈-C₁₂ Triglyceride), zusätzlich zum Wachsester vorhanden sein. Das Gewichtsverhältnis von Gesamtphospholipid (z.B. etwa Lecithin + hydriertes Lecithin) zu Gesamtlipid (z.B. Wachsester + optionale Triglyceride) beträgt in dieser Ausführungsform bevorzugt etwa 1 : 5 bis etwa 1 : 1, vorzugsweise etwa 1 : 2.

Das Gemisch aus Phospholipid und Lipid wird beispielsweise als Schmelze mit Wasser unter einem hohen Energieeintrag dispergiert. Der hohe Energieeintrag kann durch Ultraschall oder durch Hochdruckhomogenisation erfolgen, wobei Drücke von 50000 bis 250000 Kilopascal (500 bis 2500 bar), vorzugsweise 100000 bis 150000 Kilopascal (1000 bis 1500 bar) zum Einsatz kommen. In der Wasserphase können optional weitere Zusatzstoffe, wie in der vorliegenden Beschreibung beschrieben, wie etwa Glycerol oder Verdickungsmittel (z.B. Xanthangummi und/oder Hydroxypropylmethylcellulose (Hypromellose)), vorhanden sein.

Kommerziell erhältliche Basiscremes, die auf die oben beschriebenen "hautähnlichen" Bestandteile zurückgreifen, sind in der Technik auch als DMS® Basiscremes bekannt. Die DMS® Basiscremes sind Emulsionen, die membranbildende Substanzen enthalten, und werden typischerweise durch Hochdruckhomogenisation erhalten. Als Cremegrundlage können DMS® Basiscremes, z.B. durch Apotheker oder die weiterverabeitende Kosmetikinstitute, in bestehende Formulierungen eingerührt werden, und darin die oben beschriebenen lamellaren Strukturen ausbilden (siehe z.B. Österreichische Apothekerzeitung, 56 (14), 679 (2002)).

DMS-Basiscremes enthalten als Hauptkomponenten beispielsweise Wasser, hydriertes Lecithin, Fettstoffe wie Triglyceride und Squalan, Phytosterine, z.B. aus Shea-Butter, und Feuchthaltestoffe wie z.B. Glycerin (siehe z.B. Österreichische Apothekerzeitung, 56 (14), 679 (2002)).

Die DMS® Basiszusammensetzungen können insbesondere die folgenden Bestandteile aufweisen: Caprylsäure-/Caprinsäuretriglycerid, Sheabutter, Squalan, Ceramid 3, hydriertes Lecithin, Palm Glyceride, Persea Gratissima, Palmöl (Elaesis Guineensis). Als Stabilisatoren können z.B. Alkohole oder Glykole wie z.B. Pentylenglykol, Caprylylglykol oder Mischungen davon in den DMS® Zusammensetzungen eingesetzt werden. Bevorzugt wird bei den DMS-Konzentraten und bei den erfindungsgemäßen Formulierungen auf weitere (nicht körperidentische) übliche Hilfsstoffe wie Duftstoffe, Farbstoffe, komedogene Lipide (z.B. Mineralöle) und physiologische Emulgatoren verzichtet, da diese potenziell sensibilisierend sind und zu Irritationen der Haut führen können.

Die Zusammensetzung verschiedener, kommerziell erhältlicher DMS®-Basiscremes wird in der WO 2008/155389 auf Seiten 22 bis 23 beschrieben, deren Offenbarung hiermit durch Verweis aufgenommen wird.

Eine bevorzugte DMS-Grundlage umfasst die folgende Zusammensetzung: Wasser, hydriertes Lecithin, Caprylsäure/Caprinsäuretriglycerid, Pentylenglykol, Butyrospermum Parkii Butter, Glycerin, Squalan, Ceramid 3. Diese ist erhältlich von der Fa. KUHS unter dem Namen Probiol.

In bevorzugten Ausführungsformen, in denen die mindestens eine membranbildende Substanz beispielsweise in Form einer DMS-Basiscreme verwendet wird, umfasst diese folgende Inhaltsstoffe:
- Caprylsäure-/Caprinsäuretriglycerid, Sheabutter, Squalan, Ceramid 3, hydriertes Lecithin, sowie Pentylenglykol; oder
- Caprylsäure-/Caprinsäuretriglycerid, Sheabutter, Squalan, Ceramid 3, hydriertes Lecithin sowie Alkohol, oder
- Caprylsäure-/Caprinsäuretriglycerid, Sheabutter, Squalan, Ceramid 3, hydriertes Lecithin, Persea Gratissima sowie Caprylylglykol, oder
- Caprylsäure-/Caprinsäuretriglycerid, Sheabutter, Squalan, Ceramid 3, hydriertes Lecithin, Palmglycerid, Elaesis Guineensis, sowie Pentylenglykol, oder
- Caprylsäure-/Caprinsäuretriglycerid, Butyrospermum Parkii, Squalan, Ceramid 3, hydriertes Lecithin sowie Pentylenglykol, oder
- hydriertes Lecithin, Caprylsäure/Caprinsäuretriglycerid, Pentylenglykol, Butyrospermum Parkii Butter, Glycerin, Squalan, Ceramid 3.

Schaumformulierungen gemäß der vorliegenden Erfindung, die mindestens eine membranbildene Substanz enthalten, ermöglichen aufgrund des resultierenden lamellaren Membranaufbaus und der daraus resultierenden strukturellen Ähnlichkeit zum Aufbau der interzellulären lamellaren Lipidstruktur der epidermalen Lipide, insbesondere des *Stratum corneums,* eine verbesserte Pflegewirkung der Formulierung. Durch den analogen Aufbau der lamellaren Struktur der Haut wird eine leichtere Integration der Membran in die Haut erzielt. Die Integration führt ebenfalls zu einer Verbesserung, insbesondere der Stabilisierung und der Wiederherstellung, der Hautbarriere. Eine intakte Hautbarriere schützt die Haut vor zu hohem Feuchtigkeitsverlust. Eine Verbesserung der Hautbarriere kann ebenfalls eine verbesserte Hautglättung bewirken und den "wash-out" Effekt verringern, wodurch vorteilhaft ein besserer Langzeiteffekt im Vergleich zu herkömmlichen Schaumformulierungen erzielt wird.

### d) Anwesenheit herkömmlicher Emulgatoren

In einer Ausführungsform ist die Emulsion frei von folgenden Verbindungen:
- Carboxylate, wie z.B. Natriumstearat, Aluminiumstearat;
- Sulfate, wie z.B.Na-Dodecylsulfat, Na-Cetylstearylsulfat, Na-Laurylethersulfat
- Sulfonate, wie z.B. Na-Dioctylsulfosuccinat;
- quartäre Ammoniumverbindungen, wie z.B. Cetyltrimethylammoniumbromid, Benzalkoniumbromid;
- Pyridinium-Verbindungen, wie z.B. Cetylpyrdiniumchlorid;
- Betainen, wie z.B. Betainmonohydrat;
- Macrogolfettsäureester, wie z.B. Macrogol-30-Stearat;
- Glycerolfettsäureester, wie z.B. Glycerolmonostearat, Glycerolmonooleat, Glycerolmonoisostearat, Partialglyceride, mittelkettige
- Polyoxyethylensorbitan¬fettsäureester, wie z.B. Tween®, Polyoxyethylen-(20)-sorbitanmonostearat;
- Sorbitanfettsäureester, wie z.B. Sorbitanlaurat, Sorbitanmonooleat, Sorbitanmonopalmitat, Sorbitanmonostearat, Sorbitantristearat, Sorbitansesquioleat;
- Saccharosefettsäureester, wie z.B. Saccharosemonostearat; Saccharosedistearat, Sacharosecocoat;
- Macrogolfettalkoholether, wie z.B. Cetomacrogol 1000, Macrogolcetostearylether, Macrogololeylether, Lauromacrogol 400;
- Sterinalkohole, wie z.B. Cholesterol, Wollwachs, acetylierter Wollwachs, hydrierter Wollwachs, Wollwachsalkohole;
- Macrogolglycerolfettsäureester, wie z.B. Macrogol-1000-glycerol-monooleat, Macrogol-1000-glycerol-monostearat, Macrogol-1500-glycerol-triricinoleat, Macrogol-300-glyceroltris(hydroxystearat), Macrogol-5-Glycerol-stearat, Macrogolglycerolhydroxystearat;
- Polyglycerolfettsäurester, wie z.B. Triglyceroldiisostearat.

In einer weiteren Ausführungsform ist die Emulsion frei von wasserlöslichen, herkömmlichen Emulgatoren mit einem HLB-Wert von etwa 10.

In einer bevorzugten Ausführungsform umfasst die Schaumformulierung eine im Wesentlichen emulgatorfreie Emulsion. In einer besonders bevorzugten Ausführungsform umfasst die Schaumformulierung eine emulgatorfreie Emulsion.

### e) Emulgator-Copolymer

In weiteren bevorzugten Ausführungsformen umfasst die Emulsion ferner mindestens ein grenzflächenaktives, ionisches Polymer mit einem Molekulargewicht von größer als 5000 g/mol, wobei das ionische Polymer ein Copolymer ist, das als Monomereinheiten ein ionisches Monomer (M1), und mindestens ein weiteres Monomer enthält. Im Folgenden werden derartige Polymere auch als "Emulgator-Copolymer" bezeichnet.

Das mindestens eine Emulgator-Copolymer ist bevorzugt wasserlöslich oder wasserquellbar, besonders bevorzugt wasserquellbar. "Wasserquellbar" bedeutet im Rahmen der vorliegenden Erfindung, dass das Polymer bei Kontakt mit Wasser unter Volumenzunahme hydratisiert.

Bevorzugt ist das mindestens eine Emulgator-Copolymer in der Emulsion in einer Menge von ungefähr 0,01 bis ungefähr 5 Gew.-%, bevorzugt von ungefähr 0,01 bis ungefähr 1 Gew.-%, mehr bevorzugt von ungefähr 0,01 bis ungefähr 0,5 Gew.-%, ganz besonders bevorzugt von ungefähr 0,01 bis ungefähr 0,1 Gew.-% (jeweils bezogen auf das Gesamtgewicht der Emulsion ohne Treibgas) enthalten.

Enthält das Emulgator-Copolymer ein weiteres Monomer, so liegt ein Bipolymer vor, bei zwei weiteren Monomeren ein Terpolymer, usw.. Im Sinne der vorliegenden Erfindung sind Bipolymere, Terpolymere, Quaterpolymere, usw. alle von dem Begriff Copolymer umfasst.

Das mindestens eine weitere Monomer unterscheidet sich von dem ionischen Monomer (M1). Bevorzugt besitzt das mindestens eine weitere Monomer eine andere Polarität als das ionische Monomer (M1).

Das mindestens eine weitere Monomer wird vorzugsweise ausgewählt aus der Gruppe bestehend aus ionischen Monomeren, nicht-ionischen Monomeren, und Mischungen davon. Besonders bevorzugt umfasst das mindestens eine weitere Monomer mindestens ein nicht-ionisches Monomer.

### aa) Ionisches Monomer (M1):

Das ionische Monomer (M1) ist vorzugsweise anionisch, kationisch oder zwitterionisch, besonders bevorzugt anionisch.

Vorzugsweise enthält das ionische Monomer (M1) freie, partiell neutralisierte oder vollständig neutralisierte saure funktionelle Gruppen. Ein Monomer enthaltend freie saure funktionelle Gruppen wird deswegen als ionisches Monomer verstanden, weil die sauren funktionellen Gruppen entweder bei der Herstellung des Copolymers oder bei der Herstellung der Öl-in-Wasser Emulsion zumindest partiell neutralisiert werden können.

Die sauren funktionellen Gruppen sind vorzugsweise ausgewählt aus der Gruppe bestehend aus Sulfonsäuregruppen, Carbonsäuregruppen, Phosphorsäuregruppen, Phosphonsäuregruppen und Mischungen davon.

In einer bevorzugten Ausführungsform wird das ionische Monomer (M1) ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze, Ammoniumsalze oder Alkanolammoniumsalze; oder als Anhydrid vorliegen können, und Mischungen davon.

Dabei ist das ionische Monomer (M1) bevorzugt eine Acrylamidoalkylsulfonsäure, wie beispielsweise 2-Acrylamido-2-methyl-propansulfonsäure. Besonders bevorzugt liegt die Acrylamidoalkylsulfonsäure partiell oder vollständig neutralisiert als Alkalimetall-, Erdalkalimetall-, Ammonium- oder Alkanolammoniumsalz vor, besonders bevorzugt als Natrium- oder Ammoniumsalz, ganz besonders bevorzugt als Ammoniumsalz.

Besonders bevorzugt ist das ionische Monomer (M1) eine Acrylamidoalkylsulfonsäure der allgemeinen Formel (1), 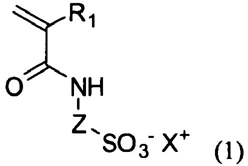 wobei R₁ ausgewählt ist aus der Gruppe bestehend aus Wasserstoff, Methyl oder Ethyl, Z ein (C₁-C₈)-Alkylen ist, das unsubstituiert oder mit einem oder mehreren (C₁-C₄)-Alkylresten substituiert sein kann, und X⁺ ausgewählt ist aus der Gruppe bestehend aus H⁺, einem Alkalimetallion, einem Erdalkalimetallion, einem Ammoniumion, einem Alkanolammoniumion, oder Mischungen davon. Vorzugsweise wird X⁺ dabei ausgewählt aus der Gruppe bestehend aus H⁺, Na⁺, NH₄⁺, oder Mischungen davon.

In einer besonders bevorzugten Ausführungsform ist das ionische Monomer (M1) 2-Acrylamido-2-methyl-propansulfonsäure (AMPS, 2-Methyl-2-[(1-oxo-2-propenyl)amino]-1-propansulfonsäure), die die chemische Formel (2) besitzt, 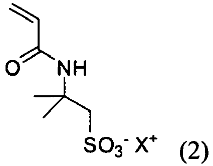 und als freie Säure (X⁺ ist gleich H⁺), oder partiell oder vollständig neutralisiert in Form ihrer Salze (der Acryloyldimethyltaurate, X⁺ ist ein Kation außer H⁺, z.B. ein Alkalimetallion wie Na⁺, ein Erdalkalimetallion, wie (1/2) Ca²⁺, oder ein Ammoniumion, wie NHa⁺) vorliegen kann. Vorzugsweise wird X⁺ dabei ausgewählt aus der Gruppe bestehend aus H⁺, Na⁺, NH₄⁺, oder Mischungen davon. Besonders bevorzugt ist das ionische Monomer (M1) Natriumacryloyldimethyltaurat oder Ammoniumacryloyldimethyltaurat (X⁺ ist gleich Na⁺ bzw. NH₄⁺).

### bb) Weiteres Monomer:

In einer Ausführungsform umfasst das mindestens eine weitere Monomer mindestens ein nicht-ionisches Monomer, bevorzugt ausgewählt aus der Gruppe bestehend aus Styrolen, Chlorstyrolen, Di-(C₁-C₃₀)-Alkylaminostyrolen, Vinylchloriden, Isoprenen, Vinylalkoholen, Vinylmethylethern, (C₁-C₃₀)-Carbonsäurevinylestern, bevorzugt Vinylacetaten und Vinylpropionaten; Acrylsäureestern, Methacrylsäureestern, Maleinsäureestern, Fumarsäureestern, Crotonsäureestern; insbesondere lineare und verzweigte (C₁-C₃₀)-Alkylester der Acrylsäure, Methacrylsäure, Maleinsäure, Fumarsäure und Crotonsäure; lineare und verzweigte (C₁-C₃₀)-Hydroxyalkylester der Acrylsäure, Methacrylsäure, Maleinsäure, Fumarsäure und Crotonsäure; ethoxylierte (C₁-C₃₀)-Alkylester der Acrylsäure, Methacrylsäure, Maleinsäure, Fumarsäure und Crotonsäure mit von 1 bis 40 Ethylenoxideinheiten; Acrylamiden, insbesondere N,N-Di-(C₁-C₃₀)-Alkylacrylamiden, Methacrylamiden, insbesondere N,N-Di-(C₁-C₃₀)-Alkylmethacrylamiden, cyclischen und linearen N-Vinylcarbonsäureamiden mit einer Kohlenstoffkette von 2 bis 9 Kohlenstoffatomen, bevorzugt N-Vinylpyrrolidon; und Mischungen davon.

In einer bevorzugten Ausführungsform umfasst das mindestens eine weitere Monomer mindestens ein nicht-ionisches Monomer ausgewählt aus der Gruppe bestehend aus linearen und verzweigten (C₁-C₆)-Hydroxyalkylestern der Acrylsäure oder Methacrylsäure, bevorzugt Hydroxyethylacrylat; ethoxylierten (C₁-C₃₀)-Alkylestern der Acrylsäure oder Methacrylsäure mit von 1 bis 40 Ethylenoxideinheiten, bevorzugt Beheneth-25-Methacrylat; N,N-Di-(C₁-C₆)-Alkylacrylamiden, bevorzugt N,N-Dimethylacrylamid, cyclischen und linearen N-Vinylcarbonsäureamiden mit einer Kohlenstoffkette von 2 bis 9 Kohlenstoffatomen, bevorzugt N-Vinylpyrrolidon, und Mischungen davon.

Das mindestens eine weitere Monomer kann auch mindestens ein ionisches Monomer umfassen, bevorzugt ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze oder Ammoniumsalze; oder als Anhydrid vorliegen können, und Mischungen davon. In einer bevorzugten Ausführungsform umfasst das mindestens eine weitere Monomer eine Acrylsäure, die partiell oder vollständig neutralisiert in Form ihres Alkalimetallsalzes, Erdalkalimetallsalzes oder Ammoniumsalzes vorliegt. Besonders bevorzugt umfasst das mindestens eine weitere Monomer dabei Natriumacrylat.

Das mindestens eine grenzflächenaktive, ionische Polymer ist vorzugsweise ausgewählt aus der Gruppe bestehend aus Acryloyldimethyltaurat/ Vinylpyrrolidon-Copolymer, Natriumacrylat/ Acryloyldimethyltaurat/ Dimethylacrylamid-Crosspolymer, Hydroxyethylacrylat/ Natriumacryloyldimethyltaurat-Copolymer, Natriumacrylat/ Natriumacryloyldimethyltaurat-Copolymer, und Mischungen davon.

### cc) Bevorzugte Emulgator-Copolymere

Ein besonders geeignetes Emulgator-Copolymer ist beispielsweise Natriumacrylat/ Natriumacryloyldimethyltaurat-Copolymer, insbesondere in Form des unter dem Namen 8885MP2 (Sepinov EG-P) von der Firma Seppic erhältlichen Produkts. Ein weiteres besonders geeignetes Emulgator-Copolymer ist Natriumacrylat/ Acryloyldimethyltaurat/ Dimethylacrylamid-Crosspolymer, insbesondere in Form des unter dem Namen 8732MP (Sepinov P88) von der Firma Seppic erhältlichen Produkts. Ein weiteres besonders geeignetes Emulgator-Copolymer ist Hydroxyethylacrylat/ Natriumacryloyldimethyltaurat-Copolymer, insbesondere in Form des unter dem Handelsnamen Sepinov^{™} EMT 10 von der Firma Seppic vertriebenen Produkts.

Ein besonders geeignetes Emulgator-Copolymer ist Acryloyldimethyltaurat/ Vinylpyrrolidon-Copolymer, bevorzugt Ammoniumacryloyldimethyltaurat/ Vinylpyrrolidon-Copolymer, insbesondere in Form des unter dem Handelsnamen Aristoflex® AVC vertriebenen Produkts.

In einer besonders bevorzugten Ausführungsform umfasst das mindestens eine Emulgator-Copolymer Acryloyldimethyltaurat/ Vinylpyrrolidon-Copolymer mit der allgemeinen Formel (3) 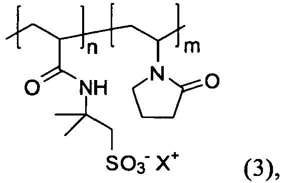 wobei X⁺ Na⁺ oder NH₄⁺ ist und n und m ganze Zahlen sind, die unabhängig voneinander zwischen 1 bis 10.000 variieren. Vorzugsweise liegt das Polymer dabei als ein statistisches Copolymer, ein Block-Copolymer oder ein Propf-Copolymer, besonders bevorzugt als ein statistisches Copolymer vor.

Das mindestens eine Emulgator-Copolymer wird bevorzugt in präneutralisierter Form verwendet, wobei es vorzugsweise pulverförmig vorliegt.

Das Emulgator-Copolymer kann beispielsweise als statistisches Copolymer, Block-Copolymer oder Propf-Copolymer oder Mischungen davon vorliegen, wobei statistische Copolymere bevorzugt sind.

In speziellen Ausführungsformen der vorliegenden Erfindung ist das Emulgator-Copolymer quervernetzt, wobei das quervernetzte Emulgator-Copolymer vorzugsweise von 0,001 bis 10 Gew.-%, besonders bevorzugt 0,01 bis 10 Gew.-% Vernetzungsmittel enthält.

Als Vernetzungsmittel können beispielsweise Diallyloxyessigsäure oder deren Salze, Trimethylolpropantriacrylat, Trimethylolpropandiallylether, Ethylenglykoldimethacrylat, Diethylenglykoldiacrylat, Tetraethylenglykoldiacrylat, Methylenbis(acrylamid), Divinylbenzol, Diallylharnstoff, Triallylamin, 1,1,2,2-Tetraallyloxyethan, Acrylsäureallylester, Methacrylsäureallylester, Dipropylenglykoldiallylether, Polyglykoldiallylether, Triethylenglykoldivinylether, oder Hydrochinondiallylether verwendet werden.

Copolymere, die intramolekulare Quervernetzungen enthalten, werden im Rahmen der vorliegenden Erfindung als "quervernetzte Copolymere" oder "Crosspolymere" bezeichnet.

### f) Feststoffemulgator

In weiteren bevorzugten Ausführungsformen umfasst die Emulsion ferner mindestens einen Feststoffemulgator.

Vorzugsweise ist der mindestens eine Feststoffemulgator in der Emulsion in einer Menge von mehr als 0,5 Gew.-%, besonders bevorzugt mehr als 1 Gew.-% enthalten. Insbesondere kann die Emulsion von 0,5 bis 7 Gew.-%, bevorzugt von 0,5 bis 5 Gew.-%, besonders bevorzugt von 0,5 bis 3 Gew.-% des mindestens einen Feststoffemulgators enthalten. Die Angaben werden jeweils auf das Gesamtgewicht der Emulsion ohne Treibgas bezogen.

Geeignete Feststoffemulgatoren sind partikuläre anorganische oder organische Feststoffe, die sowohl von lipophilen als auch von hydrophilen Flüssigkeiten benetzbar sind. Geeignete Vertreter sind z.B. Titandioxid, insbesondere beschichtetes Titanoxid (z.B. erhältlich von Merck KGaA unter der Bezeichnung Eusolex® T-2000), Zinkoxid (z.B. erhältlich von BASF SE unter der Bezeichnung Z-Cote Max), Siliziumdioxid, insbesondere hochdisperses Siliziumdioxid, Fe₂O₃, Veegum, Bentonit und Ethylcellulose. Ferner können Aluminiumoxid, Calciumcarbonat, Kohle, Magnesiumoxid, Magnesiumtrisilikat, kristalline Fettsäuren, kristalline Fettsäureester, kristalline Fettalkohole, Polymerlatices, z. B. Polystyrole oder Polymethacrylate, und Polymer-Pseudolatices verwendet werden. Auch Mischungen der vorgenannten Feststoffemulgatoren können verwendet werden. Bevorzugt ist der mindestens eine Feststoffemulgator ausgewählt aus der Gruppe bestehend aus kristallinen Fettsäuren, kristallinen Fettsäurealkylestern, kristallinen Fettalkoholen oder Mischungen davon.

Vorzugsweise umfasst der mindestens eine Feststoffemulgator eine kristalline Fettsäure, vorzugsweise mit einer Kettenlänge von 10 bis 40 Kohlenstoffatomen. Insbesondere ist die kristalline Fettsäure dabei eine gesättigte Fettsäure, bevorzugt ausgewählt aus der Gruppe bestehend aus Myristinsäure, Palmitinsäure, Margarinsäure, Stearinsäure und Arachinsäure oder Mischungen davon.

In einer besonders bevorzugten Ausführungsform umfasst der mindestens eine Feststoffemulgator Stearinsäure. Stearinsäure ist beispielsweise erhältlich von der Fa. Cognis unter dem Namen Cutina FS 45.

Ferner kann der mindestens eine Feststoffemulgator einen kristallinen Fettalkohol, bevorzugt mit einer Kettenlänge von 10 bis 40 Kohlenstoffatomen umfassen. Insbesondere ist der kristalline Fettalkohol dabei ein gesättigter Fettalkohol, bevorzugt ausgewählt aus der Gruppe bestehend aus Myristylalkohol, Cetylalkohol, Heptadecanol, Stearylalkohol, Cetylstearylalkohol, Eicosanol, oder Mischungen davon.

### g) Wasserphase

Die Wasserphase kann kosmetische Hilfsstoffe enthalten, z.B. niedere Alkohole (z.B. Ethanol, Isopropanol), niedere Diole oder Polyole sowie deren Ether (z.B. Propylenglykol, Glycerin, Butylenglykol, Hexylenglykol und Ethylenglykol), Schaumstabilisatoren und Verdickungsmittel.

Geeignete Verdickungsmittel sind polymere Verdickungsmittel, die teilweise wasserlöslich oder zumindest in Wasser dispergierbar sind und in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie die Beweglichkeit des Wassers einschränken. Geeignete Polymere sind:
- Abgewandelte Naturstoffe, wie Celluloseether (z.B. Hydroxypropylcelluloseether, Hydroxyethylcellulose und Hydroxypropylmethylcelluloseether);
- Natürliche Verbindungen, wie z.B. Xanthan, Agar-Agar, Carrageen, Polyosen, Stärke, Dextrine, Gelatine, Casein;
- Synthetische Verbindungen wie z.B. Vinylpolymere, Polyether, Polyimine, Polyamide und Derivate der Polyacrylsäure; und
- Anorganische Verbindungen wie z.B. Polykieselsäure und Tonmineralien.

Bevorzugt enthält die Emulsion mindestens ein Verdickungsmittel ausgewählt aus der Gruppe bestehend aus Hydroxypropylmethylcellulose, Xanthan Gummi, Natriumpolyacrylat, und Mischungen davon. Besonders bevorzugt enthält die Emulsion Xanthan Gummi und/oder Natriumpolyacrylat als Verdickungsmittel. Xanthan Gummi ist beispielsweise von der Fa. Kelco unter dem Namen Keltrol® CG erhältlich. Natriumpolyacrylat ist beispielsweise von der Fa. Cognis unter dem Namen Cosmedia SP erhältlich.

Die Emulsion enthält bevorzugt von 0,01 bis 1,5 Gew.-% Verdickungsmittel (bezogen auf das Trockengewicht des Verdickungsmittels und das Gesamtgewicht der Emulsion ohne Treibgas). Besonders bevorzugt sind 0,02 bis 1,0 Gew.-% Verdickungsmittel. Ganz besonders bevorzugt sind 0,02 bis 0,5 Gew.-% Verdickungsmittel.

In einer bevorzugten Ausführungsform enthält die Emulsion von 0,01 bis 1,0 Gew.-%, vorzugsweise von 0,01 bis 0,5 Gew.-% Xanthan Gummi, und/oder von 0,01 bis 1,0 Gew.-%, vorzugsweise von 0,01 bis 0,5 Gew.-% Natriumpolyacrylat (jeweils bezogen auf das Trockengewicht des Verdickungsmittels und das Gesamtgewicht der Emulsion ohne Treibgas).

### h) Wirkstoffe

Die Emulsion kann auch einen oder mehrere Wirkstoffe umfassen. Der gegebenenfalls enthaltene Wirkstoff kann unter allen oberflächig auf der Haut applizierbaren Wirkstoffen und Mischungen dieser gewählt werden. Der Wirkstoff kann kosmetisch oder pharmazeutisch wirken. Entsprechend erhält man kosmetische oder dermatologische (als Medizinprodukt oder Arzneimittelprodukt einzusetzende) Schaumformulierungen. Ferner kann die Formulierung zum Schutz der Haut vor Umwelteinflüssen dienen. Der Wirkstoff kann natürlich oder synthetisch sein. Die Gruppe der Wirkstoffe kann sich auch mit den anderen Inhaltstoffgruppen, wie z.B. der Ölkomponente, oder den gegebenenfalls enthaltenen Verdickungsmitteln oder Feststoffemulgatoren, überschneiden. Beispielsweise können manche Ölkomponenten auch als Wirkstoffe dienen, wie z.B. Öle mit mehrfach ungesättigten Fettsäuren, oder Feststoffemulgatoren, wie z.B. partikuläres Titandioxid als UV-Filter dienen können. Je nach Eigenschaftsprofil sind die Substanzen mehreren Gruppen zuzuordnen.

Insbesondere kann das in der Emulsion enthaltene mindestens eine Triterpenoid zugleich auch als Wirkstoff dienen, z.B. wenn das mindestens eine Triterpenoid Betulin oder Betulinsäure umfasst.

Wirkstoffe der erfindungsgemäßen Formulierungen werden vorteilhaft gewählt aus der Gruppe der Substanzen mit feuchtigkeitspendenden und barrierestärkenden Eigenschaften, wie z. B. Hydroviton, eine Nachbildung des NMF, Pyrrolidoncarbonsäure und deren Salze, Milchsäure und deren Salze, Glycerol, Sorbitol, Propylenglykol und Harnstoff, Substanzen aus der Gruppe der Proteine und Proteinhydrolysate wie z. B. Collagen, Elastin sowie Seidenprotein, Substanzen aus der Gruppe der Glucosaminoglucane, wie z. B. Hyaluronsäure, aus der Gruppe der Kohlenhydrate, wie z. B. Pentavitin, das in seiner Zusammensetzung dem Kohlehydratgemisch der menschlichen Hornschicht entspricht, und der Gruppe der Lipide und Lipidvorstufen wie beispielsweise die Ceramide. Weitere vorteilhafte Wirkstoffe können im Sinne der vorliegenden Erfindung ferner gewählt werden aus der Gruppe der Vitamine, wie z. B. Panthenol, Niacin, α-Tocopherol und seine Ester, Vitamin A sowie Vitamin C. Außerdem können die Wirkstoffe, gewählt aus der Gruppe der Antioxidantien z. B. Galate und Polyphenole, verwendet werden. Harnstoff, Hyaluronsäure und Pentavitin sind bevorzugte Substanzen.

Es ist ferner bevorzugt, dass als Wirkstoffe Substanzen mit hautberuhigender und regenerierender Wirkung eingesetzt werden, wie z. B. Allantoin, Nachtkerzenöl, Panthenol, Bisabolol und Phytosterole.

Vorteilhafte Wirkstoffe im Sinne der vorliegenden Erfindung sind auch Pflanzen und Pflanzenextrakte. Hierzu gehören z.B. Algen, Aloe, Arnika, Bartflechten, Beinwell, Birke, Brennessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss sowie deren Extrakte.

Die erfindungsgemäßen Zubereitungen können ferner als Wirkstoffe Antimykotika und Antiseptika/Desinfizientia synthetischen oder natürlichen Ursprungs enthalten, beispielsweise Mikrosilber.

Weitere Wirkstoffe sind Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, hyperämisierende Substanzen, Koronartherapeutika (Nitrate/Nitro-Verbindungen), Virusstatika, Zytostatika, Hormone, wundheilungsfördernde Wirkstoffe, z.B. Wachstumsfaktoren, Enzympräparate und Insektizide.

In einer bevorzugten Ausführungsform enthält die Emulsion Harnstoff. In einer anderen bevorzugten Ausführungsform enthält die Emulsion Allantoin. In einer weiteren bevorzugten Ausführungsform enthält die Emulsion Harnstoff und Allantoin, und gegebenenfalls weitere der oben genannten Wirkstoffe.

In einer weiteren bevorzugten Ausführungsform enthält die Emulsion Mikrosilber, vorzugsweise in Kombination mit Harnstoff und/oder Allantoin.

### i) Weitere Bestandteile

Die Emulsion kann außerdem optional Farbstoffe, Perlglanzpigmente, Duftstoffe/Parfum, Lichtschutzfiltersubstanzen, Konservierungsmittel, Komplexbildner, Antioxidantien und Repellentien sowie pH-Wert Regulatoren enthalten. In einer bevorzugten Ausführungsform sind die Formulierungen der Erfindung jedoch frei von Bestandteilen, die zu Irritationen der Haut führen können, insbesondere frei von Duftstoffen/Parfum, Farbstoffen und herkömmlichen Emulgatoren.

Die Emulsion kann neben den bereits oben genannten Bestandteilen weitere natürliche Fette, wie z.B. Sheabutter, Neutralöle, Olivenöl, Squalan, Ceramide und Feuchthaltesubstanzen enthalten, wie sie in der Technik üblich sind.

In einer bevorzugten Ausführungsform umfasst die Emulsion Natriumchlorid als Feuchtigkeitsspender, vorzugsweise in einer Menge von 0,1 Gew.-% bis 10 Gew.-%, besonders bevorzugt 0,5 Gew.-% bis 7 Gew.-% (bezogen auf das Gesamtgewicht der Emulsion ohne Treibgas).

Die obige Aufzählung der einzelnen Bestandteile der Emulsion soll so verstanden werden, dass einzelne Beispielkomponenten aufgrund ihrer verschiedenen Eigenschaften auch mehreren Gruppen zugeordnet werden können.

### k) Treibgase

Die Schaumforulierung enthält vorzugsweise Treibgas. Geeignete Treibgase sind z. B. N₂O, Propan, Butan und i-Butan, sowie Mischungen davon. Die fertige Schaumformulierung enthält beispielsweise von 1 bis 20 Gew.-%, von 2 bis 18 Gew.-% oder von 5 bis 15 Gew.-%, bevorzugt etwa 10 Gew.-% Treibgas. Bei der Beaufschlagung der Emulsion mit Treibgas wird (druck)verflüssigtes Treibgas verwendet.

### 3. Herstellungsverfahren

Die erfindungsgemäßen Schaumformulierungen werden durch Bereitstellen einer Emulsion, vorzugsweise vom Typ Öl-in-Wasser, und Abfüllen dieser Emulsion und gegebenenfalls Beaufschlagen mit Treibgas in einen geeigneten Behälter, vorzugsweise in einen Druckbehälter, hergestellt. Alternativ zum Treibgas und Druckbehälter kann die Emulsion auch in einen anderen Behälter abgefüllt werden, der auch ohne Treibgas dafür geeignet ist, die Emulsion als Schaum abzugeben. Solche Systeme sind dem Fachmann bekannt.
a) In einer bevorzugten Ausführungsform umfasst das Verfahren zur Herstellung der Emulsion folgende Schritte:
   1. Bereitstellen einer liquiden Ölphase
   2. Zugabe des mindestens einen Triterpenoids zu der Ölphase,
   3. Bereitstellen einer Wasserphase
   4. Homogenisieren der Wasserphase mit der triterpenoidhaltigen Ölphase.

   Die Zugabe des mindestens einen Triterpenoids zu der Ölphase gemäß Schritt 2 kann vorzugsweise durch Dispergieren des mindestens einen Triterpenoids in der Ölphase erfolgen.
b) Da die Emulsion neben dem mindestens einen Triterpenoid eine Ölphase umfasst, die mindestens eine membranbildende Substanz enthält, die in der Schaumformulierung eine lamellar angeordnete Membran ausbildet, können insbesondere die in der WO 2008/155389 beschriebenen Herstellungsverfahren verwendet werden. Dabei wird das mindestens eine Triterpenoid vorzugsweise der bereitgestellten Ölphase beigefügt. Die entsprechende Offenbarung der WO 2008/155389, insbesondere der Seiten 29 bis 31, wird hiermit durch Verweis aufgenommen.
   In bevorzugten Ausführungsformen, in denen als membranbildende Substanz eine DMS® Basiscreme oder analoge Zusammensetzung verwendet wird, kann das soeben unter a) beschriebene Verfahren verwendet werden, umfassend den weiteren Schritt:
   5. Einrühren der membranbildenden Substanz in die in Schritt 4 gebildete Emulsion.
c) In solchen Ausführungsformen, in denen die Emulsion neben dem mindestens einen Triterpenoid mindestens ein Emulgator-Copolymer gemäß der vorliegenden Erfindung enthält, können beispielsweise die in der europäischen Patentanmeldung mit der Anmeldungsnummer 09 015 330.5 und mit dem Titel "Emulgatorfreie, Polymer-stabilisierte Schaumformulierungen" des Anmelders Neubourg Skin Care GmbH & Co. KG im Kapitel "Herstellungsverfahren" (Seiten 35 bis 39) beschriebenen Herstellungsverfahren verwendet werden. Dabei wird das mindestens eine Triterpenoid vorzugsweise der bereitgestellten Ölphase beigefügt. Der Inhalt der genannten europäischen Patenanmeldung 09 015 330.5, insbesondere des Kapitels zu den Herstellungsverfahren, wird hiermit durch Verweis aufgenommen.
d) In solchen Ausführungsformen, in denen die Emulsion neben dem mindestens einen Triterpenoid mindestens einen Feststoffemulgator enthält, können beispielsweise die in der WO 2008/138894 beschriebenen Herstellungsverfahren verwendet werden. Dabei wird das mindestens eine Triterpenoid vorzugsweise der bereitgestellten Ölphase beigefügt. Die entsprechende Offenbarung der WO 2008/138894, insbesondere der Seiten 16 bis 17, wird hiermit durch Verweis aufgenommen.
e) In solchen Ausführungsformen, in denen die Emulsion neben dem mindestens einen Triterpenoid:
   aa) eine Ölphase umfassend mindestens eine membranbildende Substanz, die in der Schaumformulierung eine lamellar angeordnete Membran ausbildet, und mindestens ein Emulgator-Copolymer umfasst, oder
   bb) eine Ölphase umfassend mindestens eine membranbildende Substanz, die in der Schaumformulierung eine lamellar angeordnete Membran ausbildet, und mindestens einen Feststoffemulgator umfasst, oder
   cc) mindestens ein Emulgator-Copolymer und mindestens einen Feststoffemulgator umfasst, oder
   dd) eine Ölphase umfassend mindestens eine membranbildende Substanz, die in der Schaumformulierung eine lamellar angeordnete Membran ausbildet, und mindestens ein Emulgator-Copolymer und mindestens einen Feststoffemulgator umfasst,
können für den Fachmann ersichtlich Kombinationen der unter a) bis d) beschriebenen Herstellungsverfahren angewendet werden.

### 4. Verwendungen

Die erfindungsgemäßen Schaumformulierungen können für alle kosmetischen und dermatologischen (als Medizinprodukt oder Arzneimittel) Zwecke eingesetzt werden. Zum Beispiel können die Schaumformulierungen als Hautpflegemittel oder Hautreinigungsmittel eingesetzt werden. Sie können ferner als Träger für Wirkstoffe dienen und im medizinisch-dermatologischen Bereich eingesetzt werden. Insbesondere können die Formulierungen auch als Sonnenschutzmittel eingesetzt werden, z.B. wenn sie Feststoffemulgatoren, wie beispielsweise Titandioxid, enthalten, die gleichzeitig wirksame UVA und UVB Filter sind.

### 6. Beispiele

Schaumcremes mit folgender Zusammensetzung der Emulsion wurden hergestellt:

| **Inhaltsstoff** | **INCI- oder gebräuchlicher Name** | **Prozentualer Anteil [Gew.-%]** | | |
|---|---|---|---|---|
| | | **Beispiel 1** | **Beispiel 2** | **Beispiel 3** |
| **Phase A:** | | | | |
| Cetiol V | Decyloleat | 5,00 | 5,00 | 5,00 |
| Eutanol G | Octyldodecanol | 5,00 | 5,00 | 5,00 |
| Edenor C18 98/100 | Stearinsäure | 2,50 | 2,50 | 2,50 |
| Keltrol CG | Xanthan Gummi | 0,02 | 0,02 | 0,02 |
| Arctic Birch Bark Extract | Birkenrindenextrakt | 0,30 | -- | 0,30 |
| Ritalab White Birch Bark Extract P | Birkenrindenextrakt | -- | 0,30 | -- |
| | | | | |

| **Phase B:** | | | | |
|---|---|---|---|---|
| demineralisiertes Wasser | | 63,03 | 63,03 | 61,93 |
| Natriumchlorid | | 5,50 | 5,50 | 5,50 |
| Harnstoff | | 5,50 | 5,50 | 5,50 |
| Glycerin 86%ig | | 5,00 | 5,00 | 5,00 |
| Allantoin Pulver | | 0,10 | 0,10 | 0,10 |
| Phospholipon H80 | hydriertes Phosphatidylcholin | 3,00 | 3,00 | 3,00 |
| Cosmedia SP | Natriumpolyacrylat | 0,03 | 0,03 | 0,03 |
| Aristoflex AVC | Ammoniumacryloyldimethyltaurat-VP-Copolymer | 0,02 | 0,02 | 0,02 |
| | | | | |

| **"Phase C":** | | | | |
|---|---|---|---|---|
| DMS¹ | | 5,00 | 5,00 | 5,00 |
| Nachtkerzenöl | | -- | -- | 1,00 |
| Mikrosilber | | -- | -- | 0,10 |
| | | | | |
| **Gesamt** | | **100,00** | **100,00** | **100,00** |

| | | | | |
|---|---|---|---|---|
| ¹ Die verwendete DMS-Grundlage ist Probiol von der Fa. KUHS mit folgender Zusammensetzung: Wasser, hydriertes Lecithin, Caprylsäure/Caprinsäuretriglycerid, Pentylenglykol, Butyrospermum Parkii Butter, Glycerin, Squalan, Ceramid 3. | | | | |

### Herstellung der Emulsion:

Cetiol V, Eutanol G und Edenor C18 98/100 werden im Ansatzkessel unter Rühren bei 2000 U/min auf 60 °C erwärmt. Dann werden Keltrol CG und der Birkenrindenextrakt zugegeben. Die Mischung wird auf 70 °C erwärmt und für ca. 20 Minuten weitergerührt.

Das demineralisierte Wasser wird vorgelegt und Natriumchlorid, Harnstoff, Glycerin und Allantoin zugegeben. Die Mischung wird unter Rühren bei 2000 U/min auf 60 °C erwärmt. Bei dieser Temperatur werden Phospholipon H 80, Cosmedia SP und Aristoflex AVC langsam zugegeben. Die Mischung wird auf 70 °C erwärmt und für ca. 20-30 Minuten bei 2000 U/min weitergerührt, bis eine homogene, verdickte Flüssigkeit entsteht.

Die Wasserphase (B) wird bei ca. 70 °C der Ölphase (A) zugemischt. Bei dieser Temperatur wird für ca. 20 Minuten bei 2000 U/min weitergerührt. Anschließend lässt man die Mischung unter Rühren abkühlen. Bei einer Temperatur von 35 °C und 2000 U/min wird das DMS-Konzentrat homogen eingerührt.

In Beispiel 3 wird das Mikrosilber in das Nachtkerzenöl gerührt. Diese Mischung wird ebenfalls bei 35 °C in die Emulsion eingerührt.

### Herstellung der Schaumformulierung:

Jeweils 86,5 g der Emulsion werden in Aluminiummonoblockdosen gegeben und mit 9,5 g Treibgas (Propan-Butan-Gemisch) beaufschlagt.

### Schaumerzeugung:

Ein Schaum entsteht bei der Entnahme der Schaumformulierungen der Beispiele 1 bis 3 aus der Druckgaspackung mittels eines geeigneten Ventils mit aufgesetztem Schaumapplikator. Die Struktur des Schaums bleibt für einen Zeitraum erhalten, der ausreicht, um den Schaum gleichmäßig auf der Haut zu verteilen.

## Patentansprüche

1. Schaumformulierung umfassend eine Emulsion, umfassend eine Ölphase und eine Wasserphase,
wobei die Emulsion mindestens ein Triterpenoid ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon, umfasst, und
wobei die Ölphase mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst.

2. Schaumformulierung gemäß Anspruch 1, wobei die Emulsion Betulin umfasst.

3. Schaumformulierung gemäß Anspruch 1 oder 2, wobei die Emulsion mindestens 0,05 Gew.-% des mindestens einen Triterpenoids, bevorzugt mindestens 0,08 Gew.-% des mindestens einen Triterpenoids, umfasst.

4. Schaumformulierung gemäß einem der vorangehenden Ansprüche, wobei die Emulsion von 0,05 Gew.-% bis 10 Gew.-% des mindestens einen Triterpenoids, bevorzugt von 0,08 Gew.-% bis 8 Gew.-% des mindestens einen Triterpenoids, mehr bevorzugt von 0,1 Gew.-% bis 5 Gew.-% des mindestens einen Triterpenoids und ganz besonders bevorzugt von 0,2 Gew.-% bis 3 Gew.-% des mindestens einen Triterpenoids umfasst.

5. Schaumformulierung gemäß einem der vorangehenden Ansprüche, wobei die Emulsion von 0,05 Gew.-% bis 10 Gew.-% Betulin, bevorzugt von 0,08 Gew.-% bis 8 Gew.-% Betulin, besonders bevorzugt von 0,1 Gew.-% bis 5 Gew.-% Betulin und ganz besonders bevorzugt von 0,2 Gew.-% bis 3 Gew.-% Betulin umfasst.

6. Schaumformulierung gemäß einem der vorangehenden Ansprüche, wobei von 10 Gew.-% bis 60 Gew.-% oder von 60 Gew.-% bis 100 Gew.-% der eingesetzten Menge des mindestens einen Triterpenoids in der Emulsion als partikulärer Feststoff vorliegen.

7. Schaumformulierung gemäß einem der vorangehenden Ansprüche, wobei die Emulsion weniger als 1,0 Gew.-% herkömmlicher Emulgatoren enthält, wobei herkömmliche Emulgatoren amphiphile Substanzen mit einer Molmasse < 5000 g/mol sind, die Mizellen ausbilden können.

8. Schaumformulierung gemäß einem der vorangehenden Ansprüche, wobei die mindestens eine membranbildende Substanz ein Phospholipid und ein Triglycerid umfasst.

9. Schaumformulierung gemäß einem der vorangehenden Ansprüche, wobei das Phospholipid Lecithin, vorzugsweise hydriertes Lecithin ist.

10. Schaumformulierung gemäß einem der vorangehenden Ansprüche, wobei die Emulsion ferner mindestens einen Feststoffemulgator und/oder mindestens ein grenzflächenaktives, ionisches Polymer mit einem Molekulargewicht von größer als 5000 g/mol umfasst, wobei das ionische Polymer ein Copolymer ist, das als Monomereinheiten
- ein ionisches Monomer (M1), und
- mindestens ein weiteres Monomer
enthält.

11. Schaumformulierung gemäß einem der vorangehenden Ansprüche, wobei die Schaumformulierung Treibgas enthält.

12. Verwendung einer Schaumformulierung gemäß einem der Ansprüche 1 bis 11 als Träger für einen Wirkstoff, als Hautpflegemittel, als Hautreinigungsmittel, als Sonnenschutzmittel oder zur Herstellung eines Kosmetikums, Medizinprodukts oder Arzneimittels.

13. Verwendung einer Emulsion, umfassend eine Ölphase und eine Wasserphase,
wobei die Emulsion mindestens ein Triterpenoid ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon umfasst, und
wobei die Ölphase mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst,
für die Herstellung einer Schaumformulierung.

14. Verwendung mindestens eines Triterpenoids ausgewählt aus der Gruppe bestehend aus Betulin, Betulinsäure, Betulinsäuremethylester, Betulinaldehyd, Betulonsäure, Betulonaldehyd, Lupeol, (C₁-C₆)Alkylestern der vorgenannten Säuren, oder Mischungen davon, zur Stabilisierung von Schaumformulierungen umfassend eine Emulsion,
wobei die Ölphase der Emulsion mindestens eine membranbildende Substanz umfasst, die in der Schaumformulierung eine lamellar angeordnete Membran ausbildet, wobei die mindestens eine membranbildende Substanz ein Lipid umfasst, wobei das Lipid ein Phospholipid umfasst.

15. Verwendung gemäß Anspruch 13 oder 14, wobei die Schaumformulierung eine Schaumformulierung gemäß einem der Ansprüche 1 bis 11 ist.

## Claims

1. A foam formulation comprising an emulsion, comprising an oil phase and an aqueous phase, wherein the emulsion comprises at least one triterpenoid selected from the group consisting of betulin, betulinic acid, betulinic acid methyl ester, betulinic aldehyde, betulonic acid, betulonic aldehyde, lupeol, (C₁-C₆) alkyl esters of the aforementioned acids, or mixtures thereof,
and
wherein the oil phase comprises at least one membrane-forming substance forming a lamellar arranged membrane in the foam formulation, the at least one membrane-forming substance comprising a lipid, the lipid comprising a phospholipid.

2. The foam formulation according to claim 1, wherein the emulsion comprises betulin.

3. The foam formulation according to claim 1 or 2, wherein the emulsion comprises at least 0.05 weight-% of the at least one triterpenoid, preferably at least 0.08 weight-% of the at least one triterpenoid.

4. The foam formulation according to one of the preceding claims, wherein the emulsion comprises from 0.05 weight-% to 10 weight-% of the at least one triterpenoid, preferably from 0.08 weight-% to 8 weight-% of the at least one triterpenoid, more preferably from 0.1 weight-% to 5 weight-% of the at least one triterpenoid, and particularly preferably from 0.2 weight-% to 3 weight-% of the at least one triterpenoid.

5. The foam formulation according to one of the preceding claims, wherein the emulsion comprises from 0.05 weight-% to 10 weight-% betulin, preferably from 0.08 weight-% to 8 weight-% betulin, more preferably from 0.1 weigh-% to 5 weight-% betulin, and particularly preferably from 0.2 weight-% to 3 weight-% betulin.

6. The foam formulation according to one of the preceding claims, wherein from 10 weight-% to 60 weight-% or from 60 weight-% to 100 weight-% of the utilized amount of the at least one triterpenoid are present in the emulsion as a particulate solid.

7. The foam formulation according to one of the preceding claims, wherein the emulsion contains less than 1.0 weight-% of conventional emulsifiers, conventional emulsifiers being amphiphilic substances having a molecular mass < 5000 g/mol, which can form micelles.

8. The foam formulation according to one of the preceding claims, wherein the at least one membrane-forming substance comprises a phospholipid and a triglyceride.

9. The foam formulation according to one of the preceding claims, wherein the phospholipid is lecithin, preferably hydrogenated lecithin.

10. The foam formulation according to one of the preceding claims, wherein the emulsion further comprises at least one solid emulsifier and/or at least one surface-active, ionic polymer with a molecular weight of more than 5,000 g/mol, the ionic polymer being a copolymer comprising as monomer units
- a ionic monomer (M1), and
- at least one further monomer.

11. The foam formulation according to one of the preceding claims, wherein the foam formulation contains a propellant gas.

12. Use of a foam formulation according to one of claims 1 to 11 as a carrier for an active agent, as a skin care agent, as a skin cleaning agent, as a sunscreen, or for the manufacture of a cosmetic, a medical product or a medicament.

13. Use of an emulsion, comprising an oil phase and an aqueous phase,
wherein the emulsion comprises at least one triterpenoid selected from the group consisting of betulin, betulinic acid, betulinic acid methyl ester, betulinic aldehyde, betulonic acid, betulonic aldehyde, lupeol, (C₁-C₆) alkyl esters of the aforementioned acids, or mixtures thereof, and
wherein the oil phase comprises at least one membrane-forming substance forming a lamellar arranged membrane in the foam formulation, the at least one membrane-forming substance comprising a lipid, the lipid comprising a phospholipid,
for the manufacture of a foam formulation.

14. Use of at least one triterpenoid selected from the group consisting of betulin, betulinic acid, betulinic acid methyl ester, betulinic aldehyde, betulonic acid, betulonic aldehyde, lupeol, (C₁-C₆) alkyl esters of the aforementioned acids, or mixtures thereof, for the stabilization of foam formulations comprising an emulsion, the oil phase of the emulsion comprising at least one membrane-forming substance forming a lamellar arranged membrane in the foam formulation, the at least one membrane-forming substance comprising a lipid, the lipid comprising a phospholipid.

15. Use according to claim 13 or 14, wherein the foam formulation is a foam formulation according to one of claims 1 to 11.

## Revendications

1. Formulation de mousse comprenant une émulsion, comprenant une phase d'huile et une phase aqueuse,
dans laquelle l'émulsion comprend au moins un triterpénoïde choisi à partir du groupe consistant en bétuline, acide bétulinique, ester méthylique d'acide bétulinique, aldéhyde bétulinique, acide bétulonique, aldéhyde bétulonique, lupéol, esters d'alkyle en C₁ à C₆ des acides précités, ou de mélanges de ceux-ci, et
dans laquelle la phase d'huile comprend au moins une substance formant membrane disposée de façon lamellaire dans la formulation de mousse, l'au moins une substance formant membrane comprenant un lipide, le lipide comprenant un phospholipide.

2. Formulation de mousse selon la revendication 1, dans laquelle l'émulsion comprend de la bétuline.

3. Formulation de mousse selon la revendication 1 ou 2, dans laquelle l'émulsion comprend au moins 0,05 % en poids de l'au moins un triterpénoïde, de préférence au moins 0,08 % en poids de l'au moins un triterpénoïde.

4. Formulation de mousse selon une des revendications précédentes, dans laquelle l'émulsion comprend entre 0,05 % en poids et 10 % en poids de l'au moins un triterpénoïde, de préférence entre 0,08 % en poids et 8 % en poids de l'au moins un triterpénoïde, plus préférentiellement entre 0,1 % en poids et 5 % en poids de l'au moins un triterpénoïde et encore plus préférentiellement entre 0,2 % en poids et 3 % en poids de l'au moins un triterpénoïde.

5. Formulation de mousse selon une des revendications précédentes, dans laquelle l'émulsion comprend entre 0,05 % en poids et 10 % en poids bétuline, de préférence entre 0,08 % en poids et 8 % en poids bétuline, plus préférentiellement entre 0,1 % en poids et 5 % en poids bétuline et encore plus préférentiellement entre 0,2 % en poids et 3 % en poids bétuline.

6. Formulation de mousse selon une des revendications précédentes, dans laquelle entre 10 % en poids et 60 % en poids ou entre 60 % en poids et 100 % en poids de la quantité utilisée de l'au moins un triterpénoïde sont présents dans l'émulsion comme solide particulaire.

7. Formulation de mousse selon une des revendications précédentes, dans laquelle l'émulsion comprend moins de 1,0 % en poids d'émulsifiants conventionnels, les émulsifiants conventionnels étant des substances amphiphiliques avec une masse moléculaire < 5000 g/mol, qui peuvent former des micelles.

8. Formulation de mousse selon une des revendications précédentes, dans laquelle l'au moins une substance formant membrane comprend un phospholipide et un triglycéride.

9. Formulation de mousse selon une des revendications précédentes, dans laquelle le phospholipide est de la lécithine, de préférence de la lécithine hydrogénée.

10. Formulation de mousse selon une des revendications précédentes, dans laquelle l'émulsion en outre comprend au moins un émulsifiant solide et/ou au moins un polymère ionique tensioactif avec un poids moléculaire supérieur de 5000 g/mol, le polymère ionique étant un copolymère comprenant comme des unités monomériques
- un monomère ionique (M1), et
- au moins un monomère additionnel.

11. Formulation de mousse selon une des revendications précédentes, dans laquelle la formulation de mousse comprend un gaz propulseur.

12. Utilisation d'une formulation de mousse selon une des revendications 1 à 11 comme excipient pour un agent actif, comme agent de soin pour la peau, comme agent de nettoyage pour la peau, comme filtre solaire, ou pour la fabrication d'un produit cosmétique, d'un produit médical ou d'un médicament.

13. Utilisation d'une émulsion, comprenant une phase d'huile et une phase aqueuse,
dans laquelle l'émulsion comprend au moins un triterpénoïde choisi à partir du groupe consistant en bétuline, acide bétulinique, ester méthylique d'acide bétulinique, aldéhyde bétulinique, acide bétulonique, aldéhyde bétulonique, lupéol, esters d'alkyle en C₁ à C₆ des acides précités, ou de mélanges de ceux-ci, et
dans laquelle la phase d'huile comprend au moins une substance formant membrane disposée de façon lamellaire dans la formulation de mousse, l'au moins une substance formant membrane comprenant un lipide, le lipide comprenant un phospholipide, pour la fabrication d'une formulation de mousse.

14. Utilisation d'au moins un triterpénoïde choisi à partir du groupe consistant en bétuline, acide bétulinique, ester méthylique d'acide bétulinique, aldéhyde bétulinique, acide bétulonique, aldéhyde bétulonique, lupéol, esters d'alkyle en C₁ à C₆ des acides précités, ou de mélanges de ceux-ci, pour la stabilisation de la formulations de mousses comprenant une émulsion, la phase d'huile de l'émulsion comprenant au moins une substance formant membrane disposée de façon lamellaire dans la formulation de mousse, l'au moins une substance formant membrane comprenant un lipide, le lipide comprenant un phospholipide.

15. Utilisation selon la revendication 13 ou 14, dans laquelle la formulation de mousse est une formulation de mousse selon une des revendications 1 à 11.
